(19) Europäisches Patentamt
European Patent Office
Office européen des brevets

(11) **EP 4 114 515 B1**

(12) **EUROPEAN PATENT SPECIFICATION**

(45) Date of publication and mention
of the grant of the patent:
**28.01.2026 Bulletin 2026/05**

(21) Application number: **21711324.0**

(22) Date of filing: **04.03.2021**

(51) International Patent Classification (IPC):
*A61N 2/02* (2006.01)   *A61N 2/00* (2006.01)

(52) Cooperative Patent Classification (CPC):
**A61N 2/02; A61N 2/006**

(86) International application number:
**PCT/GB2021/050536**

(87) International publication number:
**WO 2021/176219 (10.09.2021 Gazette 2021/36)**

(54) **GENERATION OF CONTROLLABLE MAGNETIC STIMULI**

ERZEUGUNG VON STEUERBAREN MAGNETISCHEN STIMULI

GÉNÉRATION DE STIMULI MAGNÉTIQUES COMMANDABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.03.2020 GB 202003206**

(43) Date of publication of application:
**11.01.2023 Bulletin 2023/02**

(73) Proprietor: **Oxford University Innovation Limited
Oxford, OX2 0JB (GB)**

(72) Inventors:
• **DENISON, Timothy**
**Oxford OX3 7DQ (GB)**
• **ROGERS, Daniel James**
**Oxford OX1 3PJ (GB)**
• **MEMARIAN SORKHABI, Majid**
**Oxford OX3 7DQ (GB)**

(74) Representative: **Murgitroyd & Company
165-169 Scotland Street
Glasgow G5 8PL (GB)**

(56) References cited:
**WO-A1-2013/131639    US-A- 6 123 658
US-A1- 2013 030 239**

**EP 4 114 515 B1**

## Description

The present invention relates to a stimulation circuit for generating magnetic stimulation for stimulation of a body organ. Magnetic stimulation is disclosed, inter alia, in US2013/030239 A1.

Magnetic stimulation, and in particular transcranial magnetic stimulation (TMS), is an important tool used by researchers to study the central and peripheral nervous systems, and by clinicians to diagnose and treat diseases such as depression, stroke and pain. The key advantage of TMS is that it is non-invasive, and carries relatively few risks for test subjects, patients and research animals [1]. TMS is also extremely versatile, in that many body organs can be stimulated. Furthermore, the choice of specific TMS stimulation parameters (e.g. timing of pulse sequences) can have either inhibitory or facilitatory effects on brain networks, including induction of long-lasting neuroplasticity [2] [3]. Repetitive TMS (rTMS) protocols, where stimulation pulses are provided in rapid succession, are also growing in popularity; but generating consecutive and constant stimuli at high rates is one of the main challenges of this method.

Current TMS technology limits the possibilities for novel stimulation paradigms in neuroscientific experiments. Many of these limitations are inherent in the physical principles by which TMS operates. For example, one of the key restrictions of current TMS systems is the pulse shape and pattern. Due to the large instantaneous power, conventional TMS devices often use LC oscillator circuits, which generate damped cosine stimuli with highly restricted stimulus shape options. The technical limitations of the existing methods may limit their clinical effectiveness, and certainly constrain their potential for TMS stimulation in research.

The rapid advance in power electronics technology has made it possible to more precisely control the peak megawatt electrical power required in TMS devices. This control is often used for the generation of near-rectangular pulses. For example, controllable TMS (CTMS) devices were introduced in 2008, 2011 and 2014 with three different architectural variations. These designs generated a near-rectangular stimulus whose pulse width is adjustable [4] [5] [6], but a large voltage drop was seen in the wide stimuli. Pulse amplitude setup was performed separately using adjunctive equipment. The use of two independent AC/DC converters (in the second and third versions) increased the complexity and cost of the system, particularly the energy rebalancing between the capacitors in the monophasic repetitive protocols. In addition, the reliance on two energy sources meant that stimulus polarity was commutated by manually changing the mechanical orientation of the stimulation coil.

To improve TMS flexibility, flexTMS was introduced by Gattinger et. al in 2012 [7]. This instrument used a single external DC source and employed a H-bridge structure as an DC/AC inverter. Several limitations of flexTMS result from its small energy storage capacitors, which cause a large voltage decay at the beginning and end of each pulse, and greatly reduce the effective pulse width. The flexTMS design is optimized for producing a single sine-shape pulse with almost constant frequency; it has only limited ability to produce different waveforms. It is important to note that the flexTMS design does not modulate the controlling switches during a pulse; they switch only once during the pulse cycle. In addition, relatively large-amplitude oscillation artefacts are seen in the switching sequence. Other designs have demonstrated more adjustable stimulus parameters, although they are generally restricted to low power and thus are only suitable for small-animal experiments [8] [9].

Therefore, there is still a need for improved magnetic stimulation systems, in particular for TMS, that provide greater flexibility in pulse generation, for example in the shape and pattern of pulses.

The inventions and its most advantageous embodiments are defined in the appended set of claims.

According to the present invention, there is provided a stimulation circuit for generating magnetic stimulation, the stimulation circuit comprising: a coil arrangement configured to apply magnetic stimulation to a body organ; a DC supply circuit arranged to provide a DC supply; a DC/AC inverter arranged to receive the DC supply and generate a stimulation signal which is supplied to the coil arrangement, wherein the DC/AC inverter comprises a bridge inverter stage comprising plural switch modules connected in a bridge arrangement between input terminals at which the DC supply is received and output terminals for supplying the stimulation signal; and a driver circuit arranged to supply pulse width modulation control signals to the switch modules that are selected to control the DC/AC inverter to generate the stimulation signal with pulse width modulation (PWM) of voltage.

The present stimulation circuit represents a novel magnetic stimulation architecture, which uses power-electronic modulation techniques to construct arbitrary, high-power waveforms. The stimulation circuit is particularly suited for use in TMS. Stimulus pulses generated by the present system expand beyond conventional damped cosine or near-rectangular pulses, and approach an arbitrary waveform. Specifically, pulse width modulation is used to drive the switch modules of the bridge inverter stage in order to create a highly-controllable AC output waveform from a DC source. The modulation technique enables control of the waveform, frequency, and amplitude of the stimulus. PWM provides the ability to synthesize an AC output stimulus pulse for magnetic stimulation with variable frequency and amplitude from the DC input voltage source. Such waveforms promise functional advantages over the waveforms generated by current-generation TMS systems for clinical neuroscience research and treatment.

In an embodiment, the driver circuit is arranged to supply pulse width modulation control signals to the switch modules that are selected to control the DC/AC inverter to generate the stimulation signal having a controllable desired waveform, frequency and amplitude by pulse width modulation of voltage. The driver circuit may generate the pulse width modulation control signals based on a reference signal that represents the desired waveform, frequency and amplitude. The desired waveform, frequency and amplitude may be user-specified. The resulting stimulation signal may have a waveform matching or approximately matching that of the reference signal, allowing the providing an arbitrarily selectable stimulation signal.

Each switch module comprises at least one switch. The presence of switches in the bridge arrangement allows the directionality of current flow to the coil arrangement to be controlled.

In an embodiment, the driver circuit comprises current-balancing resistors connecting each switch to the driver circuit. Current-balancing resistors protect the switches from damage due to too much current being supplied. They can also be used to balance current between switches in the same switch module to prevent uneven switching of switches in the same switch modules.

In an embodiment, the driver circuit comprises transient voltage suppression diodes connected across terminals of each switch. The voltage suppression diodes prevent damage to the switches due to transients from stray inductance, overshoot, and electromagnetic interference that can occur at high switching speeds, or other sources of transient voltages.

In an embodiment, the switch modules comprise diodes connected in anti-parallel across each switch. This allows residual current from the coil arrangement to be recovered even when the switches are open.

In an embodiment, each switch is a semiconductor switch, optionally an insulated-gate bipolar transistor. Semiconductor switches can be controlled using lower voltages and currents than other switch types such as relays. Insulated-gate bipolar transistors are particularly suited to providing very high switching speeds at high voltages and currents.

In an embodiment, the switch modules comprise snubbing circuits connected in parallel with each switch. Snubbing circuits protect the switches from transient voltages due to stray inductances in the inverter circuit or DC supply circuit when the stimulation circuit is operated at high switching speeds.

The bridge inverter stage comprises plural switch modules connected in an H-bridge arrangement between the DC supply circuit and output terminals. An H-bridge arrangement allows the polarity of voltage to the coil arrangement to be switched by controlling the switch modules, which allows greater flexibility in the waveforms that can be injected into the coil arrangement.

In an embodiment, the control signals are selected to control the DC/AC inverter to supply a stimulation signal with unipolar pulse width modulation of voltage. Unipolar pulse width modulation provides greater flexibility than bipolar modulation, and generates reduced electromagnetic interference due to the smaller voltage steps in the modulated signal.

The DC/AC inverter comprises a cascade of bridge inverter stages, the input terminals of each bridge inverter stage being connected to the DC supply to receive the DC supply, the output terminals of the bridge inverter stages being connected in series for supplying the stimulation signal with multiple voltage levels. The cascade increases the number of available voltage levels in the stimulation signal, thereby increasing the flexibility of waveforms that can be injected into the coil arrangement.

The DC supply circuit includes a capacitive energy stage comprising plural capacitive energy storage modules, each arranged to supply the DC supply, each bridge inverter stage being connected to a respective capacitive energy storage module. Providing plural capacitive energy storage modules reduces the demand on each individual module, thereby reducing the likelihood of fluctuations in the DC supply voltage. However, this is not limitative and in general any suitable DC supply circuit may be used.

In an embodiment, the control signals are selected to control the DC/AC inverter to supply a stimulation signal having with multi-level pulse width modulation of voltage. Multi-level pulse width modulation provides greater control over the output waveform supplied by the coil arrangement.

In an embodiment, the driver circuit is arranged to generate the pulse width modulation control signals based on comparison of the waveforms of the reference signal and at least one carrier signal. By comparing the reference waveform to an appropriately-chosen carrier signal, the control signals can be easily generated without complex processing being required.

In an embodiment, the DC supply circuit comprises a rectifier for rectifying an AC supply, and a capacitive energy storage stage connected to the rectifier. This allows the DC supply circuit to be powered using AC power such as commonly available from public power grids. The capacitive energy storage stage also smooths the rectified supply to reduces voltage fluctuations in the output of the DC supply circuit.

In an alternative, the DC supply circuit may comprise a capacitive energy storage stage and a charging circuit arranged to charge the capacitive energy storage stage.

In one type of embodiment, the charging circuit may comprise a DC supply capable of controlling the charging current to the capacitive energy storage stage.

In another type of embodiment, the charging circuit may comprise a rectifier for rectifying an AC supply. In this case, the charging circuit may additionally comprise a transformer connected to the rectifier for transforming a mains supply to provide the AC supply. This allows the AC supply voltage to be appropriately chosen even if it does not match the available mains supply voltage.

In an embodiment, the pulse width modulation has an average switching frequency of at least 1 kHz, preferably at least 10 kHz. A high switching frequency allows fine control of the waveform output to the coil arrangement.

In an embodiment, the stimulation circuit further comprises an output filter arranged between the DC/AC inverter and the coil arrangement, the output filter being a low-pass filter. The output filter has a transfer function that smooths the voltage and current supplied to the coil arrangement by attenuating the high-frequency components of the stimulation signal. The output filter may be a second-order damped or un-damped LC passive filter or may have a more complex structure such as a third- or fourth-order filter. The output filter may include tuned elements, for example a variable capacitor and/or variable inductor. The output filter can eliminate or attenuate the unwanted harmonics from the generated stimulation signal. Due to the inherent low-pass filtering properties of nerve membranes, the output filter may not be necessary, depending on the specific implementation and target specification.

In an embodiment, the driver circuit is configured to apply pre-distortion to the pulse width modulation control signals, the pre-distortion chosen to correct for distortion to the stimulation signal caused by the DC/AC inverter and/or the output filter. The pre-distortion provides frequency-dependent amplitude and phase correction of the stimulation signal to compensate for any distortion introduced by the DC/AC inverter, and/or the output filter, if present.

In an embodiment, the coil arrangement has an inductance of at most 32 $\mu$H. Increasing the inductance of the coil arrangement reduces the magnitude of the injected current into the coil arrangement and limits the size of the electromagnetic interference generated by the changing currents in the coil arrangement and increases the responsiveness of the coil arrangement.

In an embodiment, the stimulation signal which has a peak current of at least 500 A. This ensures that the magnetic field generated by the coil arrangement is sufficiently large to achieve the desired body organ stimulation effect.

In an embodiment, the stimulation signal has a peak voltage of at least 200 V. This contributes to ensuring that the energy delivered to the body organ by the coil arrangement is sufficiently large to achieve the desired body organ stimulation effect.

In an embodiment, the coil arrangement comprises a circular coil. Circular coils generate a larger spread of magnetic field, and so can be used to affect a larger region of the body organ during magnetic stimulation. This may be preferred for certain treatment regimes.

In an embodiment, the coil arrangement comprises a figure-of-eight coil. Figure-of-eight coils generate a more localised magnetic field, and so can be used to generate a more intense magnetic field in a smaller region of the body organ. This may be preferred for certain treatment regimes.

The invention may be used in a method of generating magnetic stimulation, the method comprising: providing a DC supply; controlling a DC/AC inverter, which comprises a bridge inverter stage comprising plural switch modules connected in a bridge arrangement between the DC supply circuit and output terminals, with pulse width modulation control signals to generate a stimulation signal with pulse width modulation (PWM) of voltage; and supplying the stimulation signal to a coil arrangement configured to apply magnetic stimulation to a body organ.

The method of generating magnetic stimulation may be used in a method in which the generated magnetic stimulation is applied to a body organ for diagnosis or treatment of the body organ.

To allow better understanding, an embodiment of the present invention will now be described by way of non-limitative example with reference to the accompanying drawings, in which:

Fig. 1 is a diagram of a stimulation circuit for generating transcranial magnetic stimulation;
Fig. 2 shows possible designs of the coil arrangement;
Fig. 3 is a diagram of the DC/AC inverter of the stimulation circuit;
Fig. 4 is a logical diagram of a unipolar pulse width modulation (UPWM) technique for the DC/AC inverter;
Figs. 5(a) to 5(h) are diagrams of modes of operation of the DC/AC inverter;
Figs. 6(a) to 6(f) are a set of waveforms for the signals in the driver circuit and the resultant output voltage of the DC/AC inverter;
Fig. 7 is a diagram of a detailed construction of the DC supply circuit and DC/AC inverter;
Fig. 8 is a diagram of a switch module comprising two parallel IGBTs and current-balancing resistors;
Fig. 9 is a diagram of a switch module comprising two parallel IGBTs comprising transient voltage suppression (TVS) diodes;
Figs. 10(a) and 10(b) show the effect of including current-balancing resistors on the current sharing between two parallel switches in the same switch module;
Fig. 11 is a graph of the ideal and approximated behaviour of a neuronal membrane during transcranial stimulation, together with the applied pulse-width modulated coil voltage;
Figs. 12(a) to 12(h) show the results of measurements on the voltage, current, electric field and membrane potential change generated by the stimulation circuit using different stimulation signals;

Figs. 13(a) and 13(b) show results of measurements to test the voltage and current drop in the coil arrangement during repetitive pulse sequences;

Fig. 14 is a diagram of a stimulation circuit comprising a cascade of bridge inverter stages;

Figs. 15(a) and 15(b) show possible output filter architectures;

Figs. 16(a) to 16(d) show the results of measurements on the voltage and current generated by the stimulation circuit of Fig. 14 comprising a cascade of two bridge inverter stages using different stimulation signals and patterns;

Figs. 17(a) to 17(d) show a comparison of the pulse shape and neural behaviour for biphasic and monophasic stimuli produced by Magstim rapid 2, Magstim 200, 3-level PWM and 5-level PWM pulses; and

Fig. 18 shows a schematic representation of a driver circuit for generating PWM control signals.

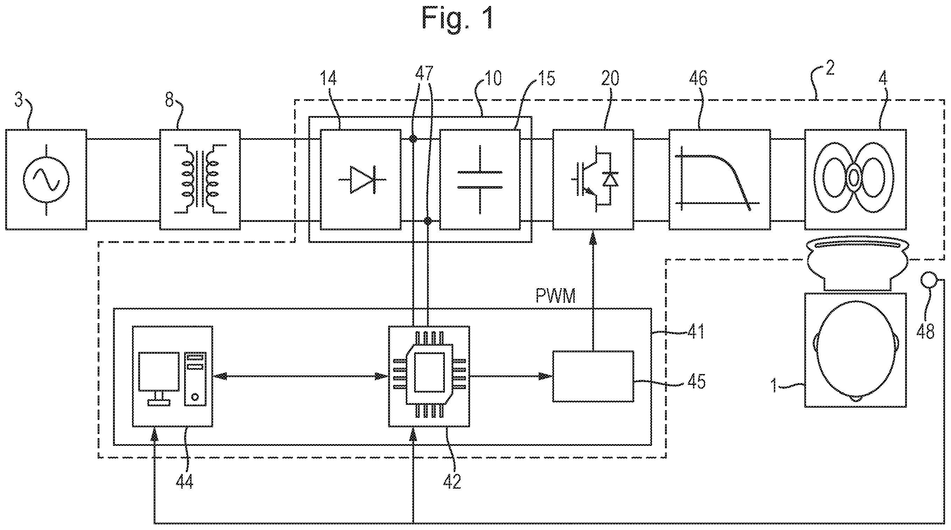

Fig. 1 shows a schematic diagram of a stimulation circuit 2 for generating magnetic stimulation, in this case transcranial magnetic stimulation (TMS). Specifically, the stimulation circuit 2 provides a programmable transcranial magnetic stimulation (PTMS) system with programmable stimulus pulses and patterns.

The stimulation circuit 2 comprises a coil arrangement 4 that may also be referred to as a stimulation coil and is configured to apply magnetic stimulation to a body organ 1, for example the brain or more generally any part of the nervous system, or a muscle. The body organ is typically of a human, but could also be of any animal.

The stimulation circuit 2 converts mains power to high-power magnetic pulses provided using the coil arrangement 4 for performing magnetic stimulation. In particular, the stimulation circuit is suited for performing TMS on a brain, but may also be used for magnetic stimulation of other body organs. In some embodiments, the coil arrangement 4 has an inductance of at most 32 $\mu$H, preferably at most 23 $\mu$H.

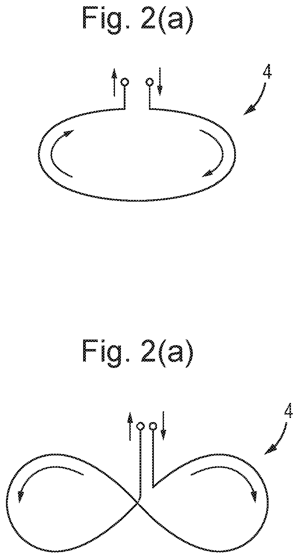

Fig. 2 shows two alternative designs of coil arrangement 4.

In Fig. 2(a), the coil arrangement comprises a circular coil. The circular coil comprises wire arranged in a single circular path, and may comprise one or more loops, or turns, in which case, the path of the wire will be helical. Where the coil comprises multiple loops, the height of the coil is typically smaller than its diameter. The magnetic field generated by a circular coil is relatively widely distributed, and so can be used to affect a larger region of the body organ during magnetic stimulation. This may be preferred for certain treatment regimes.

In Fig. 2(b), the coil arrangement 4 comprises a figure-of-eight coil. The figure-of-eight coil defines two loops around which current flows in opposite directions. For example, in Fig. 2(b) current flows anti-clockwise around the left-hand loop and clockwise around the right-hand loop. Therefore, the magnetic field generated by each loop has opposite polarity, resulting in cancellation of lower-order magnetic field components, and thereby generating a more tightly confined magnetic field at the centre of the two loops. As a result, figure-of-eight coils generate a more localised magnetic field, and can be used to generate a more intense magnetic field in a smaller region of the body organ. This may be preferred for certain treatment regimes. As for the circular coil, the figure-of-eight coil may comprise one or more loops or turns of wire. The coil arrangement 4 of Fig. 1 comprises a figure-of-eight coil.

The stimulation circuit 2 comprises a DC supply circuit 10 arranged to provide a DC supply. The DC supply circuit 10 comprises a rectifier 14 for rectifying an AC supply, and a capacitive energy storage stage 16 connected to the rectifier 14. The capacitive energy storage stage 16 in this case comprises a single capacitive energy storage module 18 that may comprise one or more DC capacitors. The rectifier 14 and capacitive energy storage stage 16 condition power from an AC supply such as the mains supply 3 to be provided to the DC/AC inverter 20. The rectifier 14 is preferably a full-wave rectifier-bridge, and converts the AC supply to a DC voltage. The DC supply circuit 10 contains an energy storage reservoir in the form of the capacitive energy storage stage 16 comprising DC capacitors.

The stimulation circuit 2 further comprises a transformer 8 connected to the rectifier 14 for transforming the mains supply 3 to provide the AC supply. Depending on the source of power for the DC supply circuit 10, it may not be necessary to provide the transformer 8 or the rectifier 14. For example, the DC supply circuit 10 may be powered using a DC power source such as a battery, or a central DC power grid of a building, if available. The transformer 8 may also be unnecessary depending on the voltage of the AC supply. However, the AC voltage required by the DC supply circuit 10 is likely to be higher than that of most mains supplies 3. Therefore, the transformer 8 is provided to step-up the mains voltage.

Thus, the rectifier 14, together with the transformer 8 (if provided) forms a charging circuit which charges the capacitive energy storage stage 16. As an alternative, these elements may be replaced by a DC supply (not shown) to act as a charging which charges the capacitive energy storage stage 16. Such a DC supply may be capable of controlling the charging current to the capacitive energy storage stage 16. Such a DC supply may include an AC/DC converter arranged to convert the mains supply 3 into DC voltage and a DC/DC converter arranged to change the voltage level of the DC voltage from the AC/DC converter.

The stimulation circuit 2 further comprises a DC/AC inverter 20 arranged to receive the DC supply and generate a stimulation signal which is supplied to the coil arrangement 4. The stimulation signal is generated such that the magnetic field from the coil arrangement 4 provides the desired form of magnetic stimulation. In some embodiments, the stimulation signal has peak current of at least 500 A, preferably at least 1000A, more preferably at least 2000A. In some embodiments, the stimulation signal has a peak voltage of at least 200 V, preferably at least 400V, more preferably at least 600V. These preferred values of voltage and current enable the coil arrangement 4 to generate sufficiently strong magnetic fields to appropriately stimulate the body organ 1, such as neural tissue in the brain.

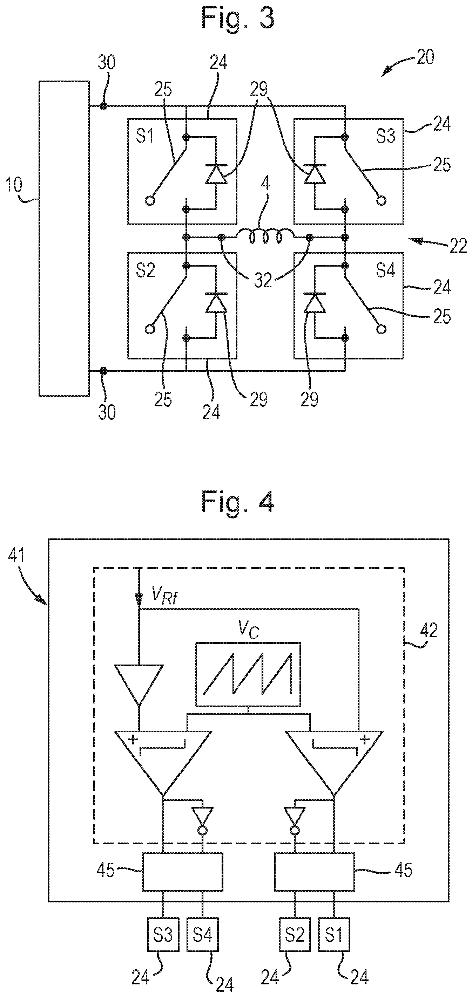

Fig. 3 is a circuit diagram showing an embodiment of the DC/AC inverter 20. The DC/AC inverter 20 comprises a bridge inverter stage 22 comprising plural switch modules 24 connected in a bridge arrangement between input terminals 30 at which the DC supply is received and output terminals 32 for supplying the stimulation signal. The bridge inverter stage 22 comprises plural switch modules 24 connected in an H-bridge arrangement between the DC supply circuit 10 and output terminals 32. However, in general, other arrangements may be used for the bridge arrangement of the switch modules of the DC/AC inverter 20, for example half-bridge, neutral point clamped inverter (NPC, Active NPC or ANPC), and flying capacitor inverter or their improved/optimized modes.

The DC/AC inverter 20 creates the PWM stimulus of the stimulation signal from the DC voltage provided by the DC supply circuit 10. The voltage generated by the H-bridge arrangement of switch modules 24 causes a current to flow in the coil arrangement 4 according to the stimulation signal to produce a time-varying magnetic field. According to the Faraday-Henry law, current flows in a conductor (including the nervous tissues of the brain) exposed to a time-varying magnetic field. This electrical current leads to the modulation of neurons and ultimately can cause the excitation/inhibition of central or peripheral nerves. Thereby the desired magnetic stimulation (e.g. TMS) effect can be achieved.

In Fig. 3, each switch module 24 comprises one switch 25. Since there are four switch modules, there are therefore four switches, labelled S1-S4. However, in general, each switch module 24 may comprise more than one switch 25, as will be discussed further below. The switches 25 used in the switch modules 24 to construct the H-bridge arrangement can be any fully-controllable switch device such as electromechanical relays or solid-state relays, but semiconductor switches, for example transistors, are preferred. Examples include bipolar junction transistors (BJTs), metal-oxide field-effect transistors (MOSFETs), insulated-gate bipolar transistors (IGBTs), or gate turn-off thyristors (GTOs), silicon carbide (Sic) switches, or gallium nitride (GaN) technologies. Generic thyristors are not preferred because they cannot be gated off. The silicon IGBT is most preferred as it is generally considered to be the most cost-effective selection for medium- to high-power applications operating in the 1-4 kV range with switching frequencies below 20 kHz [10], which are typical operating regimes for the stimulation circuit 2. Therefore, in some embodiments, each switch 25 is an insulated-gate bipolar transistor.

It is preferred that the switches 25 are able to switch quickly in order to provide sufficient resolution of the pulse-width modulation waveforms. In an embodiment, the pulse width modulation has an average switching frequency of at least 1 kHz, preferably at least 5kHz, more preferably at least 10 kHz. In order to achieve this, the switches 25 may have a switching speed of at least 1 kHz, preferably at least 5kHz, more preferably at least 10 kHz.

In Fig. 3, the DC/AC inverter 20 comprises a single H-bridge inverter stage 22. The bridge inverter stage 22 converts and controls the electromagnetic energy flow between the DC capacitors of the capacitive energy storage stage 16 and the coil arrangement 4. The transistors that provide the switches 25 in the H-bridge arrangement operate either in the fully-conducting state (near-zero resistance, ON) or fully-blocking state (near-infinite resistance, OFF) states. The OFF-ON and ON-OFF transitions occur very rapidly and so the transistors spend negligible time in a state of partial conduction.

The coil arrangement 4 connected to the output terminal 32 of the DC/AC inverter 20 is inductive, so the DC/AC inverter 20 should have an alternate path to allow the coil current to continue to flow when the switches 25 are turned off. This helps to recover coil current and prevents build-up of back electromotive force (emf) and transient voltages that could damage components of the stimulation circuit 2. For this purpose, in Fig. 3, the switch modules 24 comprise diodes 29 connected in anti-parallel across each switch 25.

The stimulation circuit of Fig. 1 further includes a driver circuit 41 arranged to supply pulse width modulation control signals to the switch modules 24 that are selected to control the DC/AC inverter 20 to generate the stimulation signal with voltage modulated by pulse width modulation. In the example shown in Fig. 1, the driver circuit 41 includes a controller 42 and optionally gate drive circuits 45. The driver circuit 41 further includes a computer 44 which controls the controller 42 and acts a control device providing overall control of the stimulation circuit 2. The computer 44 may be a conventional computer executing an appropriate computer program. Although shown as part of driver circuit 41, it is to be appreciated that the computer 44 may be an external component to which the driver circuit 41 and stimulation circuit 2 is connectable.

Fig. 4 is a diagram of an example of the driver circuit 41 arranged to provide unipolar pulse-width modulation (UPWM). PWM is a technique used extensively in electric motor drivers, solar photovoltaic (PV) inverters, and a multitude of other power conversion applications [11] [12].

The fundamental principle of PWM for magnetic stimulation is to supply a sequence of constant-amplitude, variable-width pulses of DC voltage to the coil arrangement 4 to achieve the desired stimulus. The shape and the average value of the stimulus across the stimulation coil are managed by sequentially switching the switches 25 on and off.

The desired waveform, frequency and amplitude of the stimulation signal may be specified by user input to the computer 44. The operator can select and adjust properties of the stimulation signal such as pulse shape (for example, cosinusoidal, sinusoidal, rectangular, square, ramp), pulse phase (for example, monophasic, biphasic, polyphasic), stimulus amplitude, repetitive pulse rates, and session duration. The preferred parameters are stored in the computer 44. In general, the desired waveform, frequency and amplitude may be any desired waveform of arbitrary shape. The driver circuit 41 is arranged to supply pulse width modulation control signals to the switch modules 24 that are selected to control the DC/AC inverter 20 to generate the stimulation signal having the desired waveform, frequency and amplitude by pulse width modulation of voltage.

The computer 44 supplies a reference signal $V_{Rf}$ which represents the desired stimulus waveform, frequency and amplitude to the driver circuit 41. The reference signal $V_{Rf}$ may represent the desired waveform (and frequency and amplitude) directly or in a parametrised form.

The reference signal $V_{Rf}$ is used to control the switch modules 24 of the DC/AC bridge inverter to generate a high-power imitation of the reference signal such that the stimulation signal matches or approximates the user-specified desired waveform. In particular, the driver circuit 41 is arranged to generate the pulse width modulation control signals based on the reference signal $V_{Rf}$. Thus, the driver circuit 41 converts the reference waveform $V_{Rf}$ into a train of PWM control signals $S_n$ that are supplied to the switch modules 24 to operate the switches 25 in the H-bridge arrangement of the DC/AC inverter 20.

In the example shown in Fig. 4, the controller 42 of the driver circuit 41 is arranged to generate the pulse width modulation control signals based on a comparison of the waveforms of the desired reference signal $V_{Rf}$ and at least one carrier signal $V_c$ performed in the controller 42 (although this function may alternatively be performed in the computer 44). The reference signal $V_{Rf}$ and carrier signal $V_c$ are supplied and compared by the controller 42, as shown in Fig. 4.

It is to be appreciated that any of the components of controller 42 shown in Fig. 4 may be implemented via physical circuitry or as logical blocks in the controller which may be a microprocessor executing an appropriate program. Similarly, all or part of the functions of the controller 42 may be implemented within a computer system, such as the computer 44 which accepts the user input specifying the desired waveform, frequency and amplitude.

As an alternative to generating the pulse width modulation control signals using the controller 42 based on comparison of reference signal $V_{Rf}$ and at least one carrier signal $V_c$, the driver circuit 41 may supply the pulse width modulation control signals using another source, such as a waveform generator.

In Fig. 4, the outputs of the controller 42 are connected to gate driver circuits 45 that supply the pulse width modulation control signals to the switch modules 24. However, in some embodiment, no gate driver circuits 45 may be present, and the outputs of the controller 42 may be directly connected to the switch modules 24. In an embodiment where the driver circuit 41 supplies the pulse width modulation control signals using another source, such as a waveform generator, the driver circuit 41 may not comprise a controller 42 or gate driver circuits 45, or may comprise gate driver circuits 45 but no controller 42.

The control signals are selected to control the DC/AC inverter 20 to supply a stimulation signal having a voltage that is modulated by unipolar pulse width modulation (UPWM). Therefore, the single bridge arrangement of the DC/AC inverter 20 provides a 3-level H-bridge arrangement, where the possible voltages supplied to the output terminals 32 are $+V_{DC}$, 0, and $-V_{DC}$. Unipolar PWM has the advantage that three voltage levels are available, and the changes in voltage are smaller compared to bipolar PWM, thereby producing less electromagnetic interference in any stray inductances and capacitances present in the stimulation circuit 2. Therefore, unipolar pulse width modulation is preferred, although it is not essential, and the stimulation signal could alternatively be modulated using bipolar pulse width modulation or any other switching method.

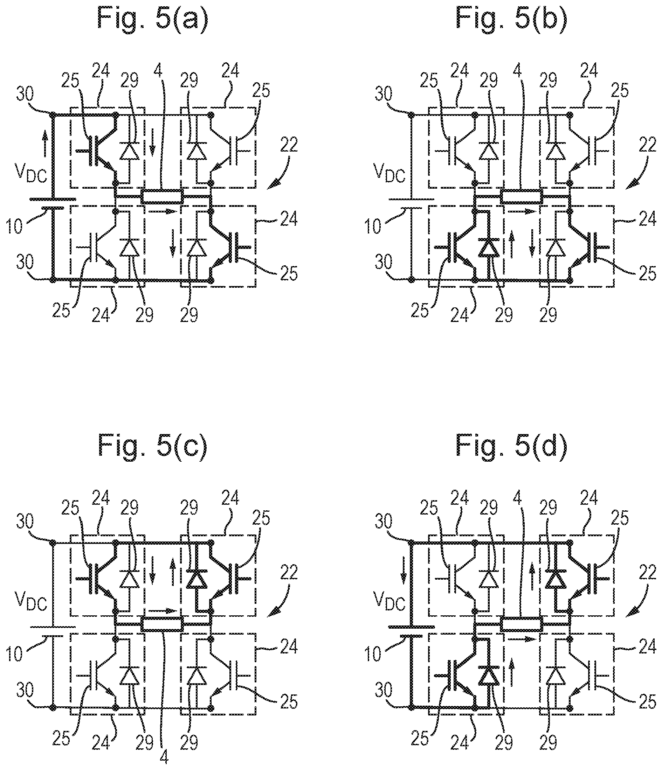

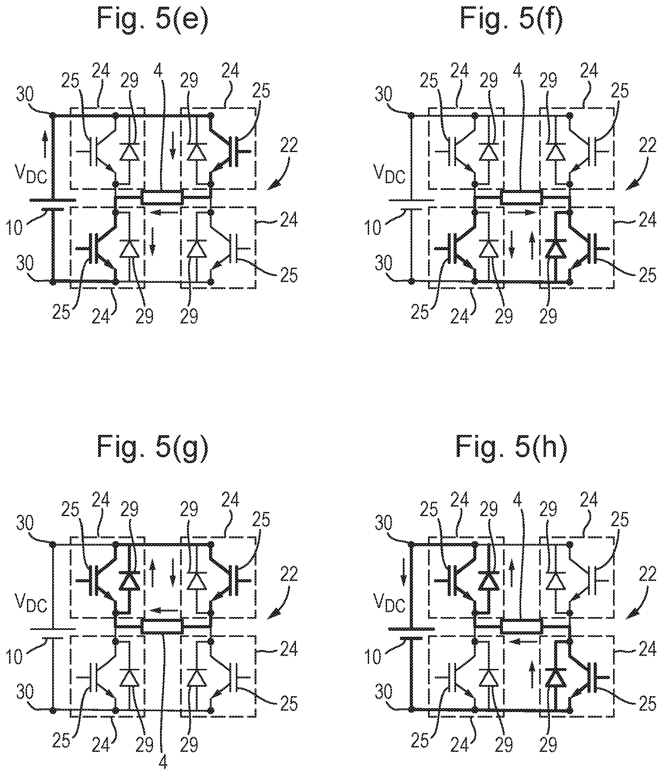

The operating modes of the H-bridge DC/AC inverter 20 for an inductive stimulation coil arrangement 4 are illustrated in Fig. 5. Fig. 5 shows a set of equivalent circuit diagrams of different modes of operation of the DC/AC inverter 20 when connected to the inductance load of the coil arrangement 4. In each case, greyed-out switch modules 24 are open (i.e. do not allow current to flow), and black switch modules 24 are closed (i.e. do allow current to flow). The battery symbol indicates the DC supply from the DC supply circuit 10. Where the battery symbol is greyed out, the DC supply circuit 10 is supplying zero voltage between the input terminals 30. The arrows indicate the direction of current flow in the DC/AC inverter 20. The different operating modes in the different diagrams are denoted as follows: (a) Mode (I); (b) Mode (II); (c) Mode (III); (d) Mode (IV); (e) Mode (V); (f) Mode (VI); (g) Mode (VII); (h) Mode (VIII).

The DC/AC inverter 20 operates in one of three types of mode: powering mode, zero mode, and regeneration mode [13], depending on the state of the switch modules 24 and the direction of the coil current, i.e. the current flowing in the coil arrangement 4. The details of the paths between the switches 25, diodes 29, and coil current are outlined in Figs. 5(a)-(h).

In powering mode, energy is transferred as current from the DC supply circuit 10 to the coil arrangement 4, and the magnitude of the coil current increases, thereby generating a magnetic field. In the powering mode, the energy is transferred from the DC capacitors of the capacitive energy storage stage 16 of the DC supply circuit 10 to the coil arrangement 4. Modes I and V shown in Figs. 5(a) and (e) respectively are both powering modes of the DC/AC inverter 20, but with opposite polarities of coil current.

In regeneration mode, the magnitude of the coil current decreases and the stored energy in the coil arrangement 4 is transferred back to the DC capacitors of the capacitive energy storage stage 16 of the DC supply circuit 10. Modes IV and VIII shown in Figs. 5(d) and (h) respectively are both regeneration modes of the DC/AC inverter 20, but with opposite polarities of coil current. Regeneration is advantageous because it recovers energy from the coil arrangement 4, and therefore results in a lower average power demand by the stimulation circuit 2 from the mains supply 3 compared to a system that reduces coil current by dissipating energy.

In zero mode, the connection between the coil arrangement 4 and the DC supply circuit 10 is cut off, and the coil arrangement 4 is shorted via the switches 25 and diodes 29 of the switch modules 24, so approximately zero voltage is applied to the coil arrangement, and the current state remains constant. Modes II, III, VI, VII, shown in Figs. 5(b), (c), (f), and (g) respectively are zero modes of the DC/AC inverter 20. In practice, the intrinsic resistances of the coil arrangement 4 and the switch module 24 will result in a small loss of energy in all modes, including zero mode, which will cause heating in these components.

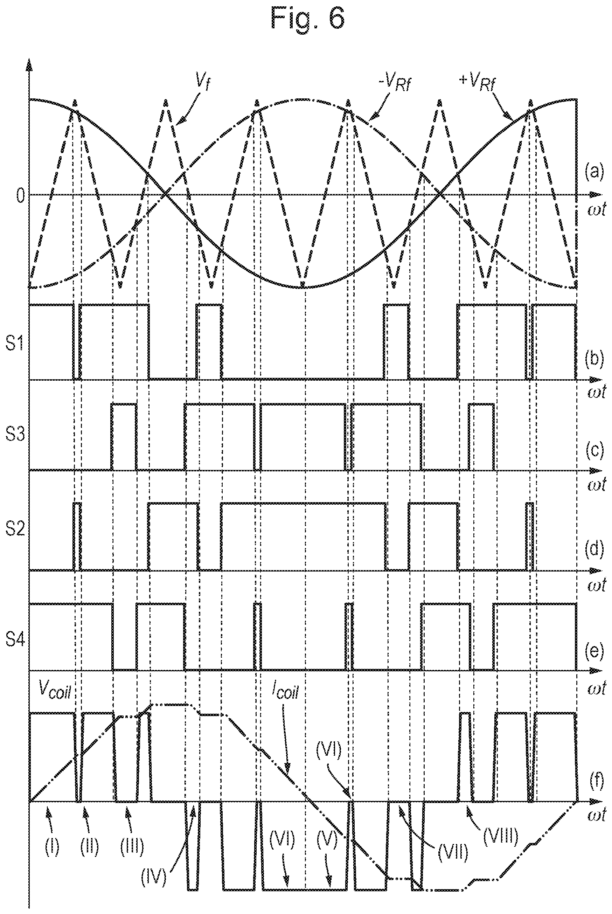

The desired coil current waveform can be produced by controlling the state of the switch modules 24 in the H-bridge arrangement using pulse-width modulation (PWM) [14]. Fig. 6 shows key waveforms in an example of operating the stimulation circuit 2 using unipolar pulse-width modulation (UPWM). A preferred method to generate the pulse width modulation control signals to control each switch module 24 in the H-bridge arrangement of the DC/AC inverter 20 is to compare a reference signal ($+V_{Rf}$) with a carrier signal (Vc), as discussed in relation to the controller 42 in Fig. 4. Generally, the carrier signal Vc is a triangular waveform and its frequency is several times that of the reference signal frequency, where the reference signal is the requested stimulus waveform to be mimicked by the PWM.

Fig. 6(a) shows a triangular waveform carrier signal Vc, with a frequency $f_c$ of 15 kHz, compared with a simple cosine reference signal ($+V_{Rf}$) having a frequency of 2.5 kHz. A cosine reference signal is used in this example for simplicity and clarity, but in general more complex waveforms could be used as the reference signal if a more complex stimulation signal were required. Also shown in Fig. 6(a) is a shifted reference signal ($-V_{Rf}$), which is identical to the reference signal $+V_{Rf}$ in magnitude and frequency, but phase-shifted by 180°. In order to generate the three-level UPWM output, the carrier signal is compared with the reference signal and the shifted reference signal. This method is called unipolar PWM (UPWM) with two phase-shifted reference signals [15].

As shown in Fig. 4, the reference signal $+V_{Rf}$ combined with the carrier signal $V_C$ is used to control switches S1 and S2 of Fig. 3, and the shifted reference signal $-V_{Rf}$ combined with the carrier signal $V_C$ is used to control switches S3 and S4 of Fig. 3. The resulting pulse width modulation control signals for each of the four switch modules 24 of Fig. 3 are shown in Figs. 6(b)-(e). As can be observed from Fig. 6, each switch module 24 is operated several times in one cycle of the reference signal. The fundamental frequency of the stimulus will be an imitation of the reference signal $+V_{Rf}$. In this UPWM switching scheme the stimulation signal voltage level changes either between 0 and $+V_{DC}$ or between 0 and $-V_{DC}$, meaning the stimulation signal has three discrete levels: 0, $-V_{DC}$, and $+V_{DC}$. Fig. 6(f) shows the stimulation signal, represented by the voltage $V_{coil}$ applied to the coil arrangement 4, and the resulting coil current. Also illustrated is the correspondence between the pulse width modulation control signals and the operating modes (I)-(VIII) of Fig. 5.

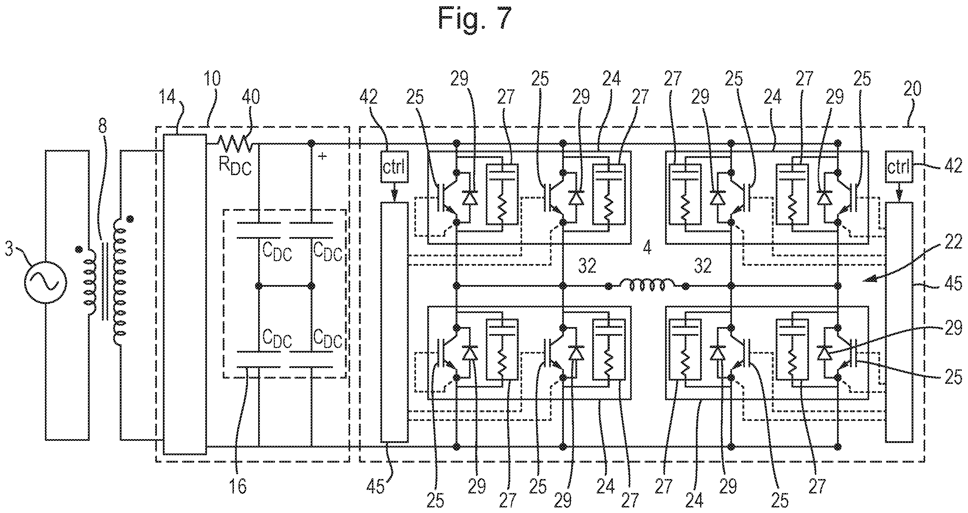

A circuit diagram of another implementation of the stimulation circuit 2 is shown in (Fig. 7). The specific components used in the implementation of Fig. 7 are provided in Table I and Table II, however the stimulation circuit 2 can be implemented with other suitable elements as well.

TABLE I: COMPONENTS OF THE SWITCH MODULE

| Component | Function | Nominal Rating | Part Number | Manufacturer |
|---|---|---|---|---|
| 28 | gate driver core | $V_{GE\,(on)}$= 15 V, $V_{GE(off)}$= -8 V<br>Gate peak current= $\pm 6$ A | 2SC0106T2A1-12 | Power Integrations |
| TVS (43) | Transient voltage suppressor (TVS) diodes | Bi-directional diode Breakdown Voltage: $\pm 19.7$ V | SMBJ16CA | Littelfuse Inc. |
| R1, R2 | Turn off and turn on resistor | 22 $\Omega$, non-inductive, carbon composition resistor $R_{G\,(on)}$= 11 $\Omega$, $R_{G\,(off)}$= 22 $\Omega$ | RCC025 22R J | Arcol |
| $R_{GE}$ | Gate- emitter resistor | 22 k$\Omega$, non-inductive, carbon composition resistor | RCC050 22K J | Arcol |
| $R_{E1}$, $R_{E1'}$ (26) | Current-balancing (feedback) resistor | 500 m$\Omega$, 20 W non-inductive | AP821 R5 J | Arcol |

8

(continued)

| Component | Function | Nominal Rating | Part Number | Manufacturer |
|---|---|---|---|---|
| D1 | Parallelize the two resistors during the turning un pulse | Extremely fast Schottky diode, Peak forward surge current: 25 A | 1N5819-E3/54 | Vishay Semiconductor |

TABLE II: KEY COMPONENTS OF THE STIMULATION CIRCUIT

| Component | Function | Nominal Rating | Part Number | Manufacturer |
|---|---|---|---|---|
| 8 | Step-up transformer | Output: 1 kV,10 A Class-E insulation | Custom manufactured | Eastern Transformers, UK |
| 14 | Full bridge diode rectifier | 1200 V, Ultrafast recovery diode $I_{FRM}$ [a] = 600 A | STTH9012TV1 | STMicroelectronics |
| $R_{DC}$ (40) | Charging resistor | 2x 47 $\Omega$, thick- film on steel 2.5 kV, 1.5 kW (parallel) | WDBR1-47RKT | TT ELECTRONICS |
| $C_{DC}$ (16) | Energy storage | 10000 $\mu$F, 500 VDC Aluminium Capacitor | ALS70A103NT500 | KEMET Electronics |
| 25 | IGBT power switch | 1.2 kV $I_{CRM}$ [b] = 1.8 kA | SEMiX603GB12E4p | Semikron |
| 29 | Free-wheeling diode (included in the IGBT module) | 1.2 kV $I_{FRM}$ [c] = 1.8 kA | | |
| 27 | Snubber capacitor | 2 $\mu$F, Polypropylene film capacitor | 205PPA122K | Illinois Capacitor |
| | Snubber resistor | 1$\Omega$, non-Inductive film resistor | AP821 1R F | Arcol |
| 4 | Stimulation coil | 15.5 $\mu$H | D70 Remote Coil | Magstim |
| Ctrl (42) | Digital controller | PWM generation resolution: 10 ns | MicroLabBox, includes Power PC Dual Core 2 GHz processor, DS1202, DS1302 I/O | dSPACE |
| a. Repetitive peak forward current, tp = 5 $\mu$s, F= 5 kHz square b. Peak current value at collector output during pulse operation c. Repetitive peak forward current of the free-wheeling diode | | | | |

In the implementation of Fig. 7, four switch modules 24 are present, each switch module 24 comprising plural switches 25 connected in parallel. Fig. 7 illustrates the case that each switch module 24 comprises two switches 25, but in general each switch module 24 could comprise any number of switches 25 connected in parallel.

The switches 25 are insulated gate bipolar transistors (IGBTs). In some embodiments, each switch module 24 may comprise more than two switches 25 connected in parallel. Using two or more switches 25 connected in parallel means that the current through the switch module 24 is shared between the switches 25. This reduces the power loss due to resistive heating, and also increases the responsiveness of the switches 25. Each switch module 24 is connected to a gate driver circuit 45, as mentioned above and discussed further below. The gate driver circuit 45 receives the pulse width modulation control signals from the controller 42. Although the controller 42 is shown twice in Fig. 7, this is merely for convenience, and both gate driver circuits 45 may be connected to the same single controller 42.

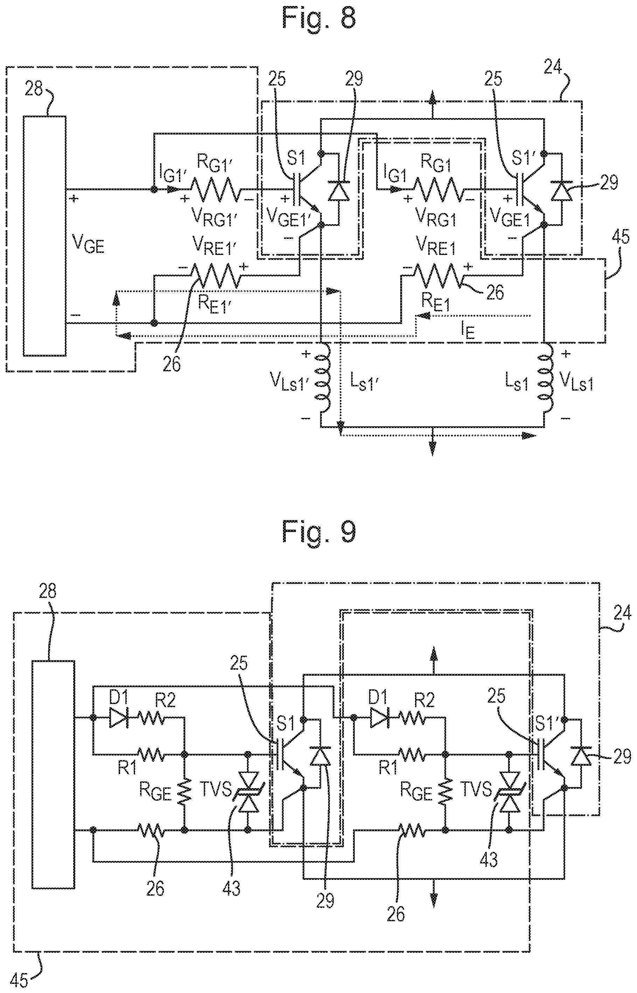

Where two or more parallel switches 25 are used in each switch module 24, and the switches 25 comprise IGBT transistors, direct connections of the IGBT emitters and their stray inductances can overload the auxiliary emitter terminals of the IGBTs. This can be illustrated using the example switch module 24 and gate driver circuit 45 shown in Fig. 8. The switch module 24 of Fig. 8 comprises two switches 25 connected in parallel. The gate driver circuit 45 comprises a gate driver core 28, which receives the pulse width modulation control signals from the controller 42. As discussed further below, the inclusion of a gate driver core 28 is advantageous when using high-power transistors such as IGBTs.

Consider the case where the two parallel switches 25 are IGBTs, and are connected to the gate driver core 28 directly with no resistance between their emitters and the gate driver core 28 ($R_{E1}$=$R_{E1'}$ = 0). In this situation, dissimilar stray inductances ($L_{s1} \neq L_{s1'}$) or different switching behaviour result in different voltage drops ($V_{Ls1} \neq V_{Ls1'}$). This voltage difference causes static and dynamic imbalances between the two emitter currents. By adding two low-value current-balancing resistors 26 to the gate driver circuit 45 between the emitters and the gate driver core 28 ($R_{E1}$, $R_{E1'} \neq 0$), this current passes through the current-balancing resistors 26 and is dissipated, helping to protect the IGBTs. The current-balancing resistors 26 also serve as a current balancer. Therefore, in an embodiment, the driver circuit 41 comprises current-balancing resistors 26 connecting each switch 25 to the driver circuit 41. Specifically, in Fig. 8, the gate driver circuit 45 comprises the current-balancing resistors 26 connected between the gate driver core 28 and the emitters of the transistors. More generally, the gate driver circuit 45 may not comprise a gate driver core 28, and the switches 25 of the switch modules 24 may be connected to the controller 42 (or a waveform generator where no controller 42 exists) without an intermediate gate driver core 28. In such embodiments, the current-balancing resistors 26 of the gate driver circuit 45 connect each switch 25 to the controller 42, specifically they connect the controller 42 to the emitters of the transistors (where the switches 25 are transistors). The driver circuit 41 may comprise multiple gate driver circuits 45, as shown in Fig. 4, and each gate driver circuit 45 may supply the pulse width modulation control signals to one or more switch modules 24. For example, in Fig. 7, each gate driver circuit 45 supplies the pulse width modulation control signals to two switch modules 24, each switch module 24 comprising two switches 25.

To further illustrate the effect of the current-balancing resistors 26, suppose S1 is turned on earlier than S1'. In this case, the voltage drops in the stray inductance of S1 ($V_{Ls1}$> $V_{Ls1'}$) and a current (IE) circulates through the current-balancing resistors 26. This current causes a voltage drop at $R_{E1}$ and $R_{E1'}$. Therefore, the gate-emitter switching voltages will be calculated according to Eqns. 1 and 2:

$$V_{GE1} = V_{GE} - V_{RG1} - V_{RE1}$$

**Equation 1**

$$V_{GE1'} = V_{GE} - V_{RG1'} + V_{RE1'}$$

**Equation 2**

The voltage drop in these current-balancing resistors 26 reduces the gate emitter switching voltage in S1 ($V_{GE1}$), which results in a slower switching speed (negative feedback). The gate-emitter switching voltage of S1' is increased, which enhances the speed of turning it on (positive feedback) [16]. This directly counteracts the difference in the time at which the two transistors switch.

Additional protection can be introduced to help avoid unwanted operating modes, i.e. states of the DC/AC inverter 20 other than those shown in Fig. 5. A leg of the bridge arrangement comprises the switch modules 24 connected to the same output terminal 32, e.g. one leg of the bridge arrangement in Fig. 3 comprises switch modules S1 and S2, and another leg comprises switch modules S3 and S4. In each leg of the bridge arrangement, the upper and lower switches 25 are complementarily controlled and should not be closed at the same time, as this would short-circuit the DC supply circuit 10 (termed a 'shoot-through condition'). Due to the asymmetric delay times of the switches 25, one switch module 24 may be switched on before another switch module 24 of the same leg is fully switched off. To prevent this, in some embodiments, the switch modules 24 may comprise dead-time circuits [17]. In addition, the high switching speeds of IGBTs are an intrinsic source of electromagnetic interference (EMI) [18]. These EMI sources are readily coupled outside the switch modules 24 by parasitic couplings with components such as the cables and printed circuit boards (PCBs) of the stimulation circuit 2. Remedial actions (such as adding electromagnetic shielding) may be necessary to prevent unwanted switching of the switch modules 24.

The structure of the switch module 24 and gate driver circuit 45 is illustrated in Fig. 9 and its components are shown in Table I. $R_{G (on)}$ and $R_{G (off)}$ are the effective gate resistances in the turn-on and turn-off states. As seen in Fig. 9, due to the presence of the diode D1, the resistance to current flow to and from the gate is different for different directions of flow, leading to different resistances in these two states. To protect the sensitive gate-emitter terminal of the switches 25 from voltage transients induced by the output of the gate driver core 28, EMI, and other temporary voltage events, transient voltage suppression (TVS) diodes 43 are incorporated into the gate driver circuit 45 connected between the base and emitter of the transistors, i.e. the driver circuit 41 comprises the transient voltage suppression diodes 43 connected across the terminals of each switch 25.

The switch module 24 is connected to the gate driver circuit 45 comprising the gate driver core 28, which is configured to receive the pulse width modulation control signals from the controller 42 and output a drive input signal to the gates of the transistors that are the switches 25 of the switch module 24. The gate driver core 28 in Fig. 9 is a Scale 2+ two-channel driver core that provides a voltage swing of +15V/-8V. The application of negative off-state voltage helps prevent the IGBT from being turned on unintentionally. For high-power transistors such as IGBTs, the use of a gate driver core 28 is advantageous, because they allow a low-voltage control signal to be amplified to the correct requirements for switching the high-power transistor. However, the gate driver core 28 is not required, and in some embodiments the driver circuit 41 may not include a gate driver circuits 45 or may not include gate driver cores 28 in any gate driver circuit 45, depending on the choice of switch 25 and other considerations. In such embodiments, the switches 25 may be directly connected to the output of the controller 42. Where gate driver cores 28 are provided in the gate driver circuit 45, each gate driver core 28 may be used to control a plurality of the switch modules 24.

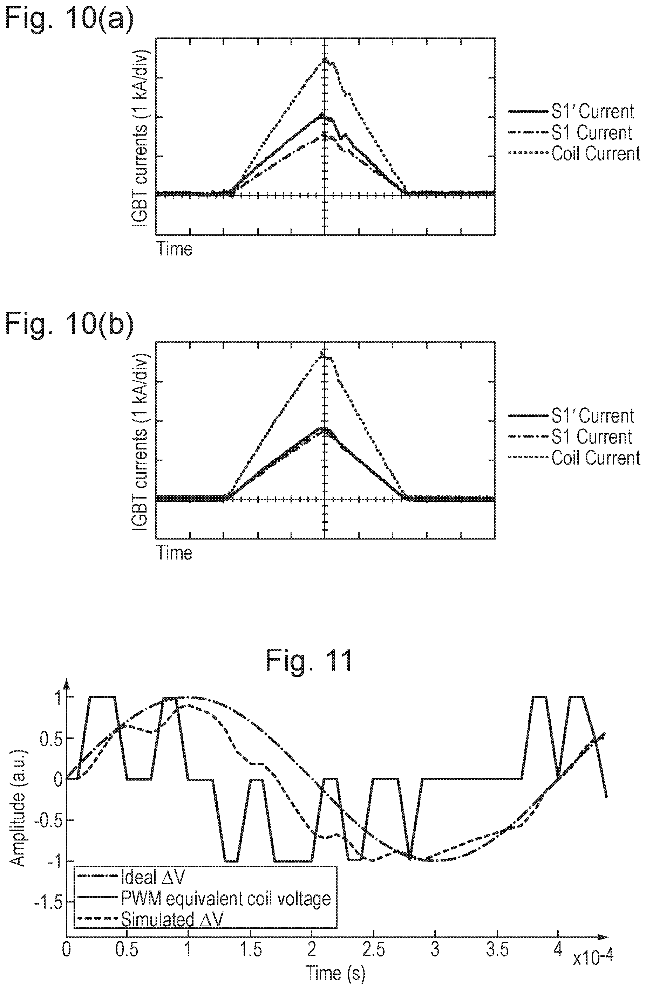

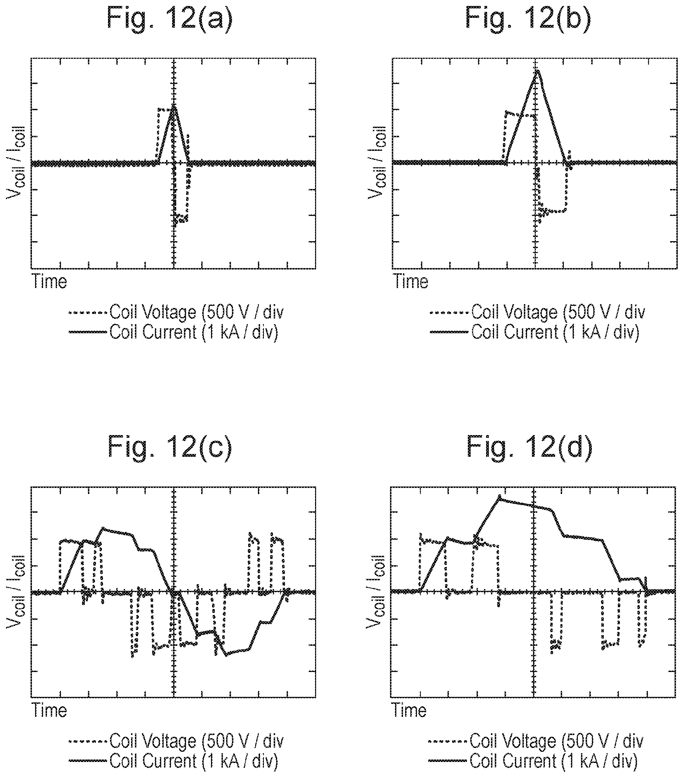

To evaluate the effect of the current-balancing resistors 26 on the current balance between the two switches 25 connected in parallel, two experiments were performed with two different values of the resistances of the current-balancing resistors 26, and are shown in Fig. 10 where each square represents 1 kA on the vertical axis and 20 μs on the horizontal axis. For both of these two experiments, a stimulus (having a waveform similar to that of Fig. 12(b)) with a positive phase of 55 μs and a negative phase of 55 μs was produced, and the currents were measured in the parallel switches S1 and S1', according to the circuit in Fig. 9. The peak coil current was $I_{coil}$= 3.2 kA. In the first experiment, the resistances of the current-balancing resistors 26 were set to zero ($R_{E1} = R_{E1'} = 0$). The results of the current measurement of each IGBT are shown in Fig. 10(a). It is seen that the lack of feedback in the emitter causes unequal distribution of current in the IGBTs. As a result of the different gate-emitter voltages in S1 and S1', arising from the different voltage drop in the parasitic inductor, the current is not shared equally between the two switches 25. The current of S1 and S1' is 60% and 40% of the total output current, respectively. By choosing non-zero resistances for the current-balancing resistors 26 and repeating the experiment ($R_{E1} = R_{E1'} = 0.5\ \Omega$, according to Table I), the results of Fig. 10(b) were obtained. The currents are almost symmetrically divided between the two parallel switches 25. Therefore, including the current-balancing resistors 26 where the switch module 24 comprises two switches 25 connected in parallel, has the advantage of more equally balancing the current between the two switches 25.

Referring again to Fig. 7, the DC supply circuit 10 comprises a full-wave diode rectifier 14, a limiter resistor 40, and a capacitive energy storage stage 16 comprising four high-voltage energy-storage DC capacitors $C_{DC}$. The size of the DC capacitors determines the maximum energy that can be delivered in one pulse. The characteristic time T of this pulse is one-quarter of the DC capacitance-coil inductance resonance period:

$$T = \frac{\pi}{2}\sqrt{LC_{tot}}$$

**Equation 3**

after which the capacitors will be fully discharged. For shorter pulses, the voltage across the DC capacitor will fall as energy is transferred to the coil arrangement 4 via the DC/AC inverter 20. The larger the DC capacitors, the smaller the voltage decay as energy is transferred, and therefore the more stable the voltage amplitude of the stimulus. For brief pulses ($t_{pulse}$<< T), the capacitor voltage can be assumed to be approximately constant. Repetitive-pulse protocols, such as theta-burst stimulation (TBS) [2] will tend to drive the determination of the DC capacitors' capacitance value because they represent a high average power. The limiter resistor ($R_{DC}$) 40 restricts the peak current to the DC capacitors during charging. In the repetitive-pulse protocols, proper heatsink connection to these resistors is likely to be required due to the high rate of pulses provided drawing significant power from the mains supply 3.

The output of the DC/AC inverter 20 is the PWM stimulation signal such as shown in Fig. 6(f). PWM produces sharp-edged rectangular voltage pulses that have significant high frequency content. This should, ideally, be removed by suitable filtering in order to produce the desired stimulus waveform. In a TMS application, there are effectively two cascaded low-pass filters naturally present in the system: Firstly, the inductance of the coil arrangement 4 provides filtering through integration of the applied PWM voltage, leading to the resulting coil current. Secondly, the neural activation response of the neural substrate in the neural tissues also acts as a low pass filter to suppress the high frequency content of the PWM stimulus and further reduces the effect of the remaining high frequency content in the magnetic field produced by the coil arrangement 4.

To better characterize the filtering properties of the neural circuit, the effect of a TMS stimulus on a neuronal membrane can be modelled using the leaky integrate-and-fire model. Considering only the subthreshold dynamics, this model describes a cell membrane as a capacitor, which is charged by an incoming current, and a resistor connected in parallel, representing the current slowly leaking across the membrane [19]. The net effect is that the cellular dynamics act approximately as a low-pass filter. When an electric field waveform $E(t)$ is applied to the neuron, the change in membrane potential ($\Delta$V) in a specific spatial coordinate is proportional to:

$$\Delta V \sim \alpha. E(t) * h(t)$$

## Equation 4

where * denotes the temporal convolution, $h(t)$ is the impulse response of a low-pass filter [20] and $\alpha$ is a constant value depending on the neural type and length, and the location of the neuron relative to the electric field [21]. The impulse response can be defined as

$$h(t) = \frac{1}{\tau_m} e^{-t/\tau_m} u(t)$$

## Equation 5

where $u(t)$ is the Heaviside step function and $\tau_m$ is the membrane time constant [20]. Assuming that the transcranial electromagnetic stimulation preferentially stimulates the axons, the membrane time constant is approximately 150 $\mu$s [4] [20] [22]. In prior work characterizing TMS systems, the same time constant has been used to predict changes of axonal membrane potential in response to stimulation [4]. To allow comparisons of the performance of the present stimulation circuit 2 with previous studies, a similar value is considered to determine nerve membrane dynamics. By connecting a passive RC filter (R represents membrane resistance and C represents membrane capacitance) with a combined time constant of 150 $\mu$s, the output of the passive RC filter can be considered as an estimate of the changes in membrane potential ($\Delta$V) [23]. Note that assuming cellular excitation rather than axonal stimulation, the time constant is an order of magnitude larger and filters the signal more strongly. Therefore, axonal excitation can be viewed as a worst-case scenario for filtering.

The approximation of the neural membrane as a low-pass filter supports the principle of using PWM for magnetic stimulation. To illustrate this, Fig. 11 shows an ideal input 2.5kHz sine wave, the PWM stimulation signal output by the stimulation circuit 2 to drive the coil arrangement 4, and the simulated membrane potential change resulting from the induced extracellular current, calculated according to Eqns. 4 and 5. The simulated membrane potential change follows the shape of the ideal sine wave, validating the use of the pulse-width modulated stimulation signal. Some residual distortion due to higher order switching harmonics is still visible.

A major advantage of the PWM approach to generating the stimulation signal is the ability to adjust the magnetic stimulation power by changing the amplitude of the reference signal, such that the stimulus amplitude can be adjusted without changing the structure of the DC/AC inverter 20, or varying the DC supply provided by the DC supply circuit 10. If the maximum amplitude of the reference signal is equal to the maximum amplitude of the carrier signal, the DC/AC inverter 20 will generate the highest voltage. In the PWM technique, the amplitude modulation index $m_a$ is [24]:

$$m_a = \frac{V_{Rf}}{V_c}$$

## Equation 6

where $V_{Rf}$ and $V_c$ are the peak values of the reference and carrier signals, respectively. The amplitude modulation index $m_a$ is commonly adjusted by varying $V_{Rf}$ while keeping $V_c$ constant. The DC/AC inverter 20 can generate a stimulation signal linearly proportional to the reference signal in the range of $0 \le m_a \le 1$ [10]. In the example of Fig. 6, the amplitude modulation index $m_a$ is 0.9. Buck-boost converter or other DC/DC and AC/DC converter structures can also be utilized to control the voltage range (not shown). This block can be located between the rectifier 14 and the capacitive energy storage stage 16 or between the transformer 8 and the rectifier 14 and connected to the controller 42 and adjusts the DC voltage amplitude with the appropriate trigger received from the controller 42. The frequency modulation index $m_f$ is given by [24]:

$$m_f = \frac{f_C}{f_{Rf}}$$

## Equation 7

where $f_C$ and $f_{Rf}$ are the frequencies of the carrier signal and the reference signal, respectively. In the example of Fig. 6, the frequency modulation index $m_f$ is 6. Clearly, whilst the stimulation signal generated by the DC/AC inverter 20 contains the desired fundamental frequency component of the reference signal, it also contains high frequency harmonics. In unipolar PWM, the first set of harmonics are the largest and are distributed around $2f_C$ [24], and then also occur at higher multiples of $f_C$. Increasing the frequency modulation index $m_f$ by increasing the carrier signal frequency $f_C$ moves the undesirable higher harmonics away from the fundamental frequency, which improves the low-pass filtering effect of the coil arrangement and neural substrate, and so provides a higher-fidelity response. However, $f_C$ is limited by the capability of the switches 25 in the DC/AC inverter 20, because increasing $f_C$ will tend to increase losses in the bridge arrangement, and will eventually lead to overheating.

The parallel connection of IGBTs as shown in Fig. 7 is necessary for increasing the current carrying capacity of the switch modules 24 beyond that of a single IGBT [25]. In the implementation of Fig. 7, two switch modules 24 are used in each leg of the DC/AC inverter 20, totalling 4 modules for the DC/AC inverter 20 bridge arrangement. Each switch module 24 comprises two switches 25, and all eight switches 25 are implemented with 1200V/1800A IGBT modules (Semikron, Germany). The tolerances in IGBT parameters, process variations, unbalanced stray inductance in the circuit, and dissimilar propagation delays from the driver circuit 41 can cause asymmetrical current balancing among parallel-connected IGBT modules within each switch module 24 under both static and dynamic operation [25]. In the implementation of Fig. 7, IGBT current-balancing resistors 26 are used as described above to enforce current sharing between the parallel-connected IGBT modules.

TMS requires the generation of very high peak power pulses (over 3 MW), which will typically exceed the current rating of individual transistors such as IGBTs. However, the average power of TMS is relatively low (a few hundred Watts in repetitive modalities). Therefore, TMS is an example of a pulsed power application which typically presents severe stress to the transistors [26] [27]. In power-electronic based systems, power semiconductor elements are one of the most fragile components [28]. A review of the effect of overcurrent on the IGBT (3.3-kV/1200-A) switches are provided by [29]. An inductive test circuit was utilized to overcurrent the switches. In that test, the parameters used were $V_{DC}$ = 2500 V, L = 100 µH, $R_G$ = 3.7 Ω and $I_{coil}$ = 3500A. More details about all the devices' physics and test circuits are available in [30]. Inside the IGBT module, physical signatures of failure were observed as a burnt-out spot on the surface at the chip level. The same results have also been reported in [31]. Burnt-out spots reduce the lifetime of the semiconductor switches and significantly enhance the risk of failure [32]. Therefore, overloading the switches, especially for medical devices, can challenge the safety of the device.

Due to the importance of repeatability and stability in medical devices, as well as operator and patient safety, all components of the stimulation circuit 2 should be strictly operated in their Safe Operating Area (SOA) to ensure reliability. To help ensure the temperature of all the switches stay within their SOA, the switch temperatures can be measured continuously using an NTC thermistor embedded in each switch module 24. In an embodiment, a real-time temperature measurement is used by the controller 42 to switch off the stimulation circuit 2 if the temperature falls outside a prescribed limit. Additionally, for this reason, switch modules 24 containing two or more parallel switches 25 are preferred to limit the IGBTs' current stress for all operating modes. The importance of overcurrent is more prominent in protocols such as PWM, which produces sequential pulses, that, in comparison to other methods of generating stimuli, can cause more thermal problems. The gate driver circuit 45 provides the transient currents required to charge/discharge the MOS-gate structure of the IGBTs, under the control of the pulse width modulation control signals from the controller 42. During the switching transient, the gate driver circuit 45 can also control the $dv/dt$ and $di/dt$ rate by the external $R_{G(off)}$ and the $R_{G(on)}$, respectively [17]. Controlling the transient behaviour of the stimulation circuit 2 helps to reduce the stress on switches 25.

In power circuits, if high currents are switched rapidly, the stray inductance in the circuit causes a voltage overshoot across the switch transistors. This overvoltage generally occurs during turn-off of the switches 25 and it is added to the DC supply voltage. For example, in the case of Fig. 7, during the turn-off state of an IGBT, the stray inductances of the commutation path can cause large over-voltages across the IGBTs. This transient voltage might exceed the maximum blocking voltage of the switches 25 and can destroy the switches [33]. Although choosing the proper length and thickness of wiring will reduce the parasitic inductance, it is not possible to completely remove it, and the internal stray inductances such as those of the IGBTs, the DC link, and the series inductance of the input will still contribute in practice. To address this problem, the switch modules 24 in the example of Fig. 7 comprise snubbing circuits 27 connected in parallel with each switch 25. The snubbing circuits 27 comprise a snubber capacitor and snubber resistor connected in series, having the values given in Table II. Alternatively, the snubber circuits 27 may comprise a resistor and a capacitor in series, with a diode in parallel with the resistor (not shown) or any active snubber architecture. The snubbing circuits 27 provide protection against voltage transients during the turn-off of the switches 25 and maintain the IGBT in the SOA [34]. By selecting a large $R_{G(off)}$ in the driver (i.e. a large effective gate resistor when switching off), the maximum overshoot voltage is also controlled, so the snubber circuit 27 requires no active compensation. A disadvantage of increasing the $R_{G(off)}$ is that this will tend to increase turn-off delay time and therefore increase switching loss.

To drive the switches 25 in each leg, a gate driver circuit 45 comprising a gate driver core 28 with separate external resistors is used. As described above, current-balancing resistors 26 help to equally share the current between switches 25 in the same switch module 24. Symmetrical wiring between the gate driver cores 28 and the switches 25, DC supply circuit 10, DC/AC inverter 20, and coil arrangement 4 helps to further reduce unbalanced current sharing.

The stimulation circuit of Fig. 7 is controlled and monitored by a controller 42 as seen in Fig. 1. The controller 42 can be a MicroLabBox commercial control system (dSPACE GmbH, Germany) or any other digital controller. The generation of high-resolution (10 ns) PWM trigger pulses enables the precise generation of stimulus pulses. The real-time operation of the controller 42 allows defining different measurements and interlocking protocols during operation of the PTMS and ensures its safe operation. The ability of the controller 42 to connect to a computer 44 and software such as MATLAB running thereon gives it a high degree of flexibility as the modulation and control protocols can be configured in the software environment, and embedded into the hardware for real-time control.

The controller output parameters include 4 PWM signals to trigger the switch modules 24, according to the logical switching block shown in Fig. 3. The input parameters of the controller 42 include the reading of the switches chip temperatures, the voltage of the coil arrangement 4, and the coil current in the coil arrangement 4. If any of these input parameters exceed the predefined safe values, the stimulation process is stopped. Specifically, these parameters are defined as an interlocking logic protocol for starting or continuing the stimulation, which guarantees that the components are kept within their SOA. In an embodiment, the controller 42 measures the voltage values of the DC supply 10 using sensors 47, as shown in Fig. 1. By controlling the pulse width modulation signals in response to voltage fluctuations, for example those originating from the mains supply 3, the stimulation signal amplitude can be tuned more efficiently.

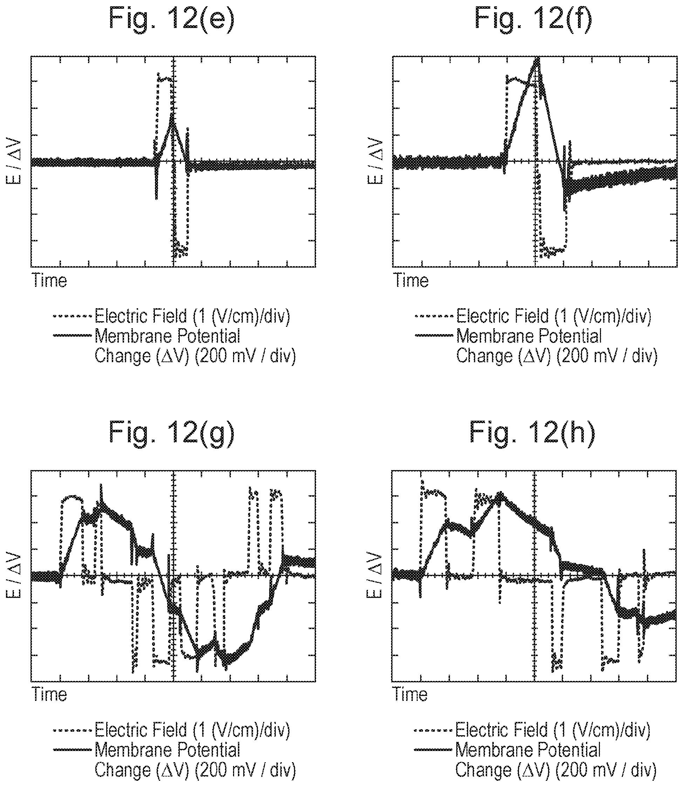

Representative measurement results of the proposed stimulation circuit 2 in Fig. 1 are shown in Fig. 12. The stimulation circuit 2 was tested with a DC supply voltage of $V_{DC}$= 1000V and peak coil current up to 3600A. The maximum transferable energy to the D70 Remote coil (Magstim, UK) making up the coil arrangement 4 was measured to be 100.4 Joules. The coil voltage and current were measured via a high- voltage differential probe (TA044, PICO TECHNOLOGY, UK) and a Rogowski current probe (I6000S FLEX-24, FLUKE, USA), respectively. All measurements were performed with a digital oscilloscope and a sampling rate of 250 MSa/ s. No bandwidth restrictions or filters have been applied to remove switching spikes. The measurements were performed for two square pulses with widths of 40 and 110 microseconds, a 2.5 kHz cosine pulse with $m_a$= 1, and a 2.5 kHz sinusoidal pulse with $m_a$=0.5. Amplitude and frequency modulation indexes were selected to achieve maximum output voltage/current value, as well as ensuring that the circuit operates in the SOA.

Figs. 12(a)-(d) show the stimulus voltage (stimulation signal) and coil current values of the stimulation circuit 2 in Fig. 1 for: (a) the 40 $\mu$s square pulse stimulus (monophasic output) (20 $\mu$s positive and 20 $\mu$s negative phase); (b) the 110 $\mu$s square pulse stimulus (monophasic output) (55 $\mu$s positive and 55 $\mu$s negative phase); (c) 2.5 kHz cosine stimulus ($m_a$= 1) (biphasic output); and (d) 2.5 kHz sine stimulus ($m_a$= 0.5) (monophasic output). Each square in Figs. 12(a)-(d) represents 500 Volts for the stimulus voltage and 1 kA for the coil current on the vertical axis, and 50 $\mu$s on the horizontal axis. The stimulus voltage and coil current are dependent on the inductance of the coil arrangement 4 (arising from the electromagnetic design of the particular coil), the frequency of the stimulation signal, and the amplitude modulation index.

The induced electric field (E) was measured with a custom-made pickup coil consisting of a single-turn rectangular winding (0.15-mm-thick copper wire). The pick-up coil dimensions were 1 cm $\times$ 35 cm, in a perpendicular position to the stimulation coil centre. The small side of the rectangular pick-up coil was located 1 mm away from the surface of the stimulation coil. The value of the induced electric field can be estimated by dividing the measured electromotive force (EMF) by the search coil width (w):

$$E \cong EMF/w$$

## Equation 8

If the search coil width is 1 centimetre (w=1 cm), the measured EMF is approximately equal to the induced electric field (E) measured in V/cm [35] [36]. To estimate the behaviour of nerves stimulated by the induced electric fields, a physiological model of the response of the neural substrate is considered, as described above.

The induced electric field and the predicted membrane potential corresponding to the stimulus voltage (stimulation signal) and coil current values of Figs. 12(a)-(d) are shown in Figs. 12(e)-(h). Specifically, these correspond to: (e) the 40 $\mu$s square pulse stimulus; (f) 110 $\mu$s square pulse stimulus; (g) 2.5 kHz cosine stimulus ($m_a$=1); and (h) 2.5 kHz sine stimulus ($m_a$= 0.5). Each square in Figs. 12(e)-(h) represents 1 volt/cm on the vertical axis for the electric field graph and 200 mV on the vertical axis for the membrane potential, and 50 $\mu$s on the horizontal axis for both. The induced electric field waveforms follow the stimulus waveforms, and the shapes of the estimated changes in membrane potential are similar to the coil current waveforms (except the phase difference in $I_{coil}$ and $\Delta V$ caused by charging/ discharging capacitors). The behaviour of the nerve tissue in the presence of the external electric field is modelled with a passive RC filter with a time constant of 150 $\mu$s similar to [4] [5].

The measurement results prove that the shorter pulses will induce smaller changes in membrane potential, and the longer pulses that have more energy will produce larger $\Delta V$. These results are consistent with a prior study [37]. To ensure that the induction electric field generated by PTMS was sufficient for nerve stimulation, some measurements were repeated on the Magstim rapid2 option 2 device (MAGSTIM Company Ltd, UK). The experimental results confirmed that the maximum induction electric field of the stimulation circuit 2 in Fig. 1 is 63% of the maximum power of the Magstim rapid2. While some references report that the average motor evoked potential (MEP) is 40.6-55% of the maximum power of some commercial TMS devices [38] [39] [40], this parameter also depends on the TMS stimulus generator, stimulation coil, gender and age of the individual [40]. The impact of this power limitation is limited to single pulse, low duty cycle experiments. For higher pulse rates, the programmable TMS achieves parity with the Magstim rapid2, as discussed below.

The oscillations visible in the voltage and electric field are due to the action of the snubber circuit 27 to compensate for transient voltages created during switch commutation. These transient oscillations are caused by stray inductors in the circuit and IGBTs, as discussed above. For transient voltage spikes, a 20% safety margin was achieved. Experimental measurements confirmed that the switch module 24 and the snubber circuit 27 were able to keep the IGBTs within their SOA throughout operation. Furthermore, Figs. 12 (a)-(d) demonstrate that the PTMS stimuli have almost the same pulse amplitude at the beginning and end of the voltage pulse. As shown in Fig. 12(b), after a pulse of 55 $\mu$s, the voltage drop is approximately 10% from the initial value. Experiments prove that the present system can generate a wide range of stimulus waveforms. By defining the required waveform using the reference signal, the operator can estimate the PWM signal required to drive the coil arrangement 4 appropriately.

To characterize the effect of PWM stimulus on coil heating, an experiment was conducted on the D70 remote coil (MAGSTIM Company Ltd, UK), which is an example of a figure-of-eight coil. The stimulation pattern was chosen as iTBS (triplet 50 Hz bursts, repeated at 5 Hz; 2 seconds on and 8 seconds off; a total of 180 pulses) and the temperature of the coil centre was measured with a thermal camera (FLIR C2, FLIR Systems, USA). The experiment was repeated with the Magstim rapid2 option 2 device (MAGSTIM Company Ltd, UK). To produce 50 Hz pulses, the maximum output power of Magstim rapid2 is limited to 50%. Therefore, the output power of the stimulation circuit 2 in Fig. 1 was set to the same power ($m_a = 0.8$). The generated waveform was also set as a cosine of 2.5 kHz ($m_f = 6$). The experiment was performed at the same ambient temperature and the coil temperature was measured at the beginning and end of the iTBS. No significant temperature difference was observed.

rTMS has become a major area of TMS research in the last decade. This technique has been studied as a novel paradigm in treatment in a variety of neurological and psychiatric disorders [41] [42] and motivates new pulse shapes and patterns. Short interstimulus intervals are conventionally generated by connecting multiple devices to a single coil. For example, to produce a train of four monophasic magnetic pulses with a time interval of 1.5-1250 ms (called quadripulse stimulation: QPS), four Magstim TMS devices are connected with a specially designed combining module [43]. Eliminating these costly solutions motivates the design of new circuits for the generation of magnetic stimuli that can provide the ability to generate consecutive pulses required for novel therapies and experiments. In therapies invoking rTMS, the time interval between pulses is typically short, but the AC/DC power converter circuits from the mains are not capable of rapidly charging capacitors. Therefore, the designed circuits must be able to recycle the energy supplied to the coil. In the present design, the high capacitance in the DC supply circuit 10 allows the DC supply voltage to remain constant during the short pulses.

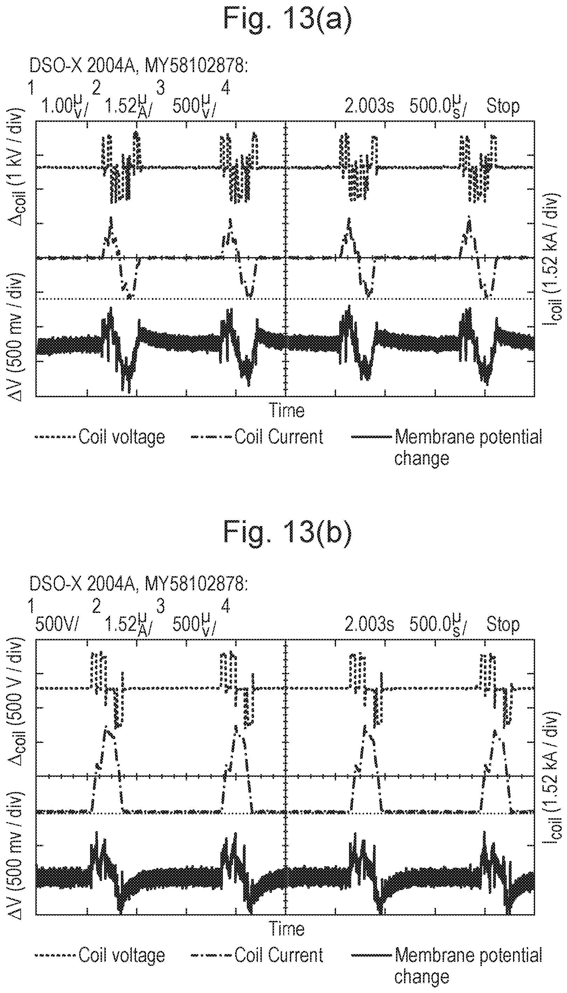

Fig. 13 illustrates the capability of the stimulation circuit shown in Fig. 1 to generate repetitive pulses, while recovering energy from the stimulation coil. Fig. 13 shows waveform parameters measured for four cosine and four sinusoidal stimuli, in quadrupoles burst modality. Fig. 13(a) shows parameters for a cosine pulses with 800$\mu$s interstimulus interval, as a biphasic stimulation. Fig. 13(b) shows parameters for sine pulses with 1000$\mu$s interstimulus interval, as a monophasic stimulation. In Fig. 13(a), each square represents 1 kV for the voltage waveforms on the vertical axis, and in Fig. 13(b), each square represents 500 V for the voltage waveforms on the vertical axis. In both Figs. 13(a) and 13(b), each square represents 1.52 kA for the coil current waveforms and 500mV for the nervous membrane voltage changes ($\Delta V$) on the vertical axis, and 500 $\mu$s on the horizontal axis. For these measurements, the oscilloscope sampling rate was 100 MSa/s.

After four pulses, the DC supply voltage attenuation caused by the parasitic resistors of the stimulation circuit 2 was less than 1%. The coil current and membrane potential variations are also less than 1%. The large time constant of the DC capacitors prevents their voltage drop and the system can produce stable stimuli. With a very short time interval, a consistent stimulus can be generated and a stable potential change in the membrane can occur. The performance of the system in the iTBS and cTBS modality was also evaluated. In this technique, three pulses repeated with an interstimulus interval of 20 milliseconds (50 Hz burst of the 3 stimuli) based on the protocol defined in [2]. The measured parameters were similar to Fig. 13. The losses occurring in the long interstimulus interval (caused by the parasitic coil path resistance) are compensated by the recharge of the DC supply from the mains supply. Energy efficiency in the TMS system becomes more critical for complex repetitive patterns and burst stimulation. The main reason for the DC voltage drops between the start and end point of the pulse is the connection of the DC capacitors to the stimulation coil and the start of the LC oscillation. At equal width pulses, any system that has a large capacitor will have a lower voltage drop, as discussed above. Similarly, for a fixed coil parasitic resistance, the discharge time constant of a small capacitor is shorter than for a larger capacitor. For a pulse with a positive phase and a width of 55 $\mu$s, the voltage decay caused by these effects in existing systems is 25% in the CTMS3 circuit [6], 89% in the Flex-TMS circuit [7]. This compares to a drop of 10% in the stimulation circuit 2. According to the results reported in [6], the pulse amplitude generated by the CTMS3 circuit for pulses of 120 $\mu$s and longer approaches zero volts. The high voltage drop of these existing systems limits their performance, as the actual pulse duration will be shorter than the presumed duration. However, in the present system, by using PWM signals, which have shorter pulse width and more pulse repetition, this voltage drop is significantly reduced.

Unipolar pulse-width modulation provides three levels of voltage that can be used in the stimulation signal. However, in some situations, it can be advantageous to provide further levels of voltage in pulse-width modulation. In particular, for applications such as TMS where the voltages and switching speeds of the PWM signals are high, providing additional voltage levels reduces the number and size of voltage steps that are needed to provide a particular average voltage, thereby reducing the high frequency content of the stimulation signal and the induced electric field.

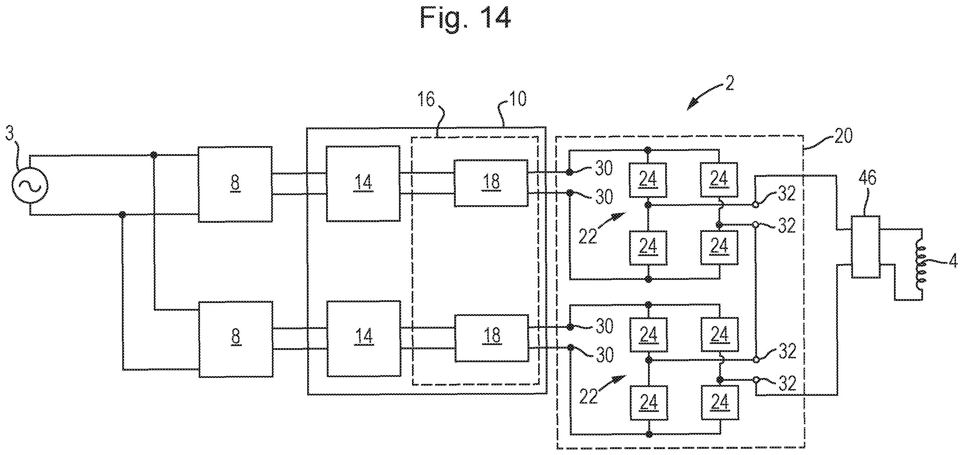

To provide additional voltage levels, multiple bridge inverter stages 22 can be provided in a cascade. Such an arrangement is shown in Fig. 14 wherein the DC/AC inverter 20 comprises a cascade of bridge inverter stages 22, the input terminals 30 of each bridge inverter stage 22 being connected to the DC supply to receive the DC supply, the output terminals 32 of the bridge inverter stages 22 being connected in series for supplying the stimulation signal with multiple voltage levels. The driver circuit 41 is not shown in Fig. 14 for simplicity. Each bridge inverter stage 22 can supply three voltage levels of $+V_{DC}$, 0, and $-V_{DC}$ as described above, meaning that the stimulation circuit 2 in Fig. 14, having two bridge inverter stages 22, can supply five voltage levels in steps of $V_{DC}$ between $+2V_{DC}$ and $-2V_{DC}$. For clarity, only two bridge inverter stages 22 are shown in and Fig. 14, but in general more bridge inverter stages 22 may be provided in the cascade to provide further voltage levels.

The DC supply circuit 10 in Fig. 14 includes a capacitive energy storage stage 16 comprising plural capacitive energy storage modules 18, each comprising one or more DC capacitors and being arranged to supply the DC supply. Each bridge inverter stage 22 is connected to a respective capacitive energy storage module 18. By providing a capacitive energy storage module 18 for each bridge inverter stage 22, the voltage decay from each capacitive energy storage module 18 and total recharging time is reduced, and so voltage fluctuations are less likely to occur, improving the consistency of the stimuli supplied by the stimulation circuit 2. In Fig. 14, the DC supply circuit 10 further comprises multiple rectifiers 14, with each capacitive energy storage module 18 of the capacitive energy storage stage 16 connected to a different rectifier 14. Similarly, the DC supply circuit 10 further comprises multiple transformers 8, with each rectifier 14 powered by a different transformer 8. Depending on the design of the stimulation circuit 2, it may not be necessary to provide rectifiers 14 or transformers 8, as discussed above. However, each bridge inverter stage 22 should be supplied from an isolated capacitive energy storage module 18, i.e. a capacitive energy storage module 18 isolated from the capacitive energy storage modules 18 to which any other bridge inverter stages 22 are connected. If the capacitive energy storage modules 18 connected to each bridge inverter stage 22 are not isolated from one another, the capacitors are effectively connected in parallel, and it would not be possible to provide additional voltage levels for multi-level PWM. Multiple separate transformers 8 (as in Fig. 14), or a single multi-winding transformer, may be used to provide galvanic isolation between the energy storage modules 18.

The control signals are selected to control the DC/AC inverter 20 to supply a stimulation signal having a voltage that is modulated by multi-level pulse width modulation. This is enabled by the provision of the additional voltage levels.

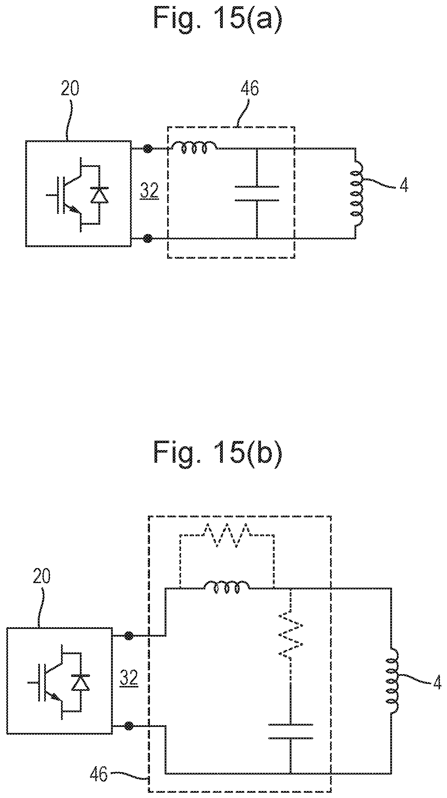

The embodiments of the stimulation circuit 2 shown in Fig. 1 and Fig. 14 further comprise an output filter 46 arranged between the DC/AC inverter 20 and the coil arrangement 4, the output filter 46 being a low-pass filter. The output filter 46 has a transfer function that smooths the stimulation signal supplied to the coil arrangement 4, and thereby the coil voltage and current, by attenuating the high-frequency components of the stimulation signal. The filter can be a second-order damped or un-damped LC passive filter or may have a more complex structure such as third- or fourth-order. The output filter may include tuned elements, for example a variable capacitor and/or variable inductor. Figs. 15(a) and 15(b) show two proposed second order LC filters that would be suitable for use as the output filter 46. Output filter 46 parameters are selected according to switching frequency, optimum stimulus frequency and other considerations. Due to the inherent low-pass filtering property of the nerve membrane, it may, depending on the specific implementation and target specification, be possible to operate without this filter.

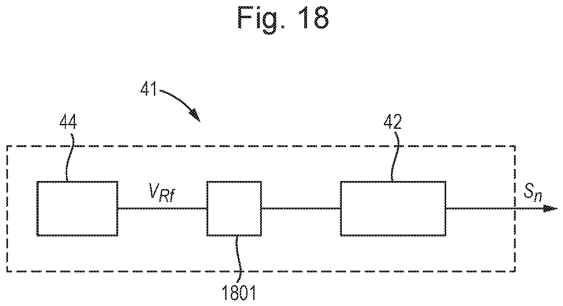

In an embodiment, the driver circuit 41 is configured to apply pre-distortion to the pulse width modulation control signals, the pre-distortion chosen to correct for distortion to the stimulation signal caused by the DC/AC inverter 20. The pre-distortion technique provides frequency-dependent amplitude and phase correction of the stimulation signal to compensate for any distortion introduced by the DC/AC inverter 20, for example due to an inverter modulation algorithm, and/or the coil arrangement 4. The pre-distortion may also compensate for distortion caused by the output filter 46 in embodiments that have an output filter 46. The pre-distortion may be generated and applied to an incoming reference signal $V_{Rf}$ by pre-distortion generator 1801, shown in Fig. 18. Pre-distortion generator 1801 may be a stand-alone circuit, or may be implemented as part of controller 42 and/or computer 44.

In Fig. 18, the reference signal $V_{Rf}$ supplied by computer 44 is passed to controller 42, via the pre-distortion generator 1801. Pre-distortion generator 1801 is configured to apply a predefined distortion (or compensation) to the reference signal $V_{Rf}$, as discussed in more detail below. The controller 42 is configured to generate and output the PWM control signals $S_n$ based on the received reference signal $V_{Rf}$, as modified by the pre-distortion generator 1801. In some embodiments, control signal generator 1803 is configured to compare the pre-distorted reference signal $V_{Rf}$ and at least one carrier signal $V_c$ as discussed above in relation to Fig. 4. The pulse width modulation control signals $S_n$ are then generated based on the comparison. In alternative embodiments, controller 42 may generate the control signals $S_n$ based on the pre-distored reference signal $V_{Rf}$ using a waveform generator, rather than by comparison to a carrier signal $V_c$.

The pre-distortion may be generated based on a comparison of the actual stimulation signal generated with the desired waveform. For example, when the pre-distortion technique is used, the pre-distortion generator 1801 (which may be controller 42 and/or the computer 44) can measure or receive a measurement of the stimulation signal using a magnetic field sensor 48, for example a search coil or a Hall effect sensor, and enhance the stimulus waveform requirements. By measuring the actual magnetic field produced by the coil arrangement 4, and comparing this to the desired waveform, appropriate pre-distortion can be applied to the pulse-width modulation control signals by the controller 42 to compensate for any distortion caused by the components of the stimulation circuit 2. The distortion correction of the stimulation signal is carried out by a closed-loop digital algorithm. By reducing the distortion created by inverter block 20 or output filter 46, magnetic stimulation devices can be made to be far more accurate.

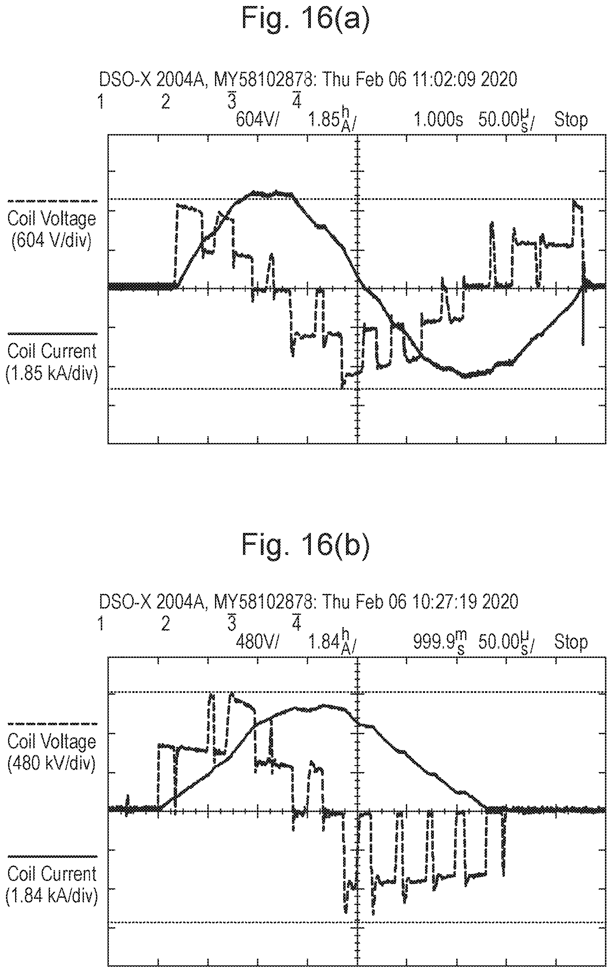

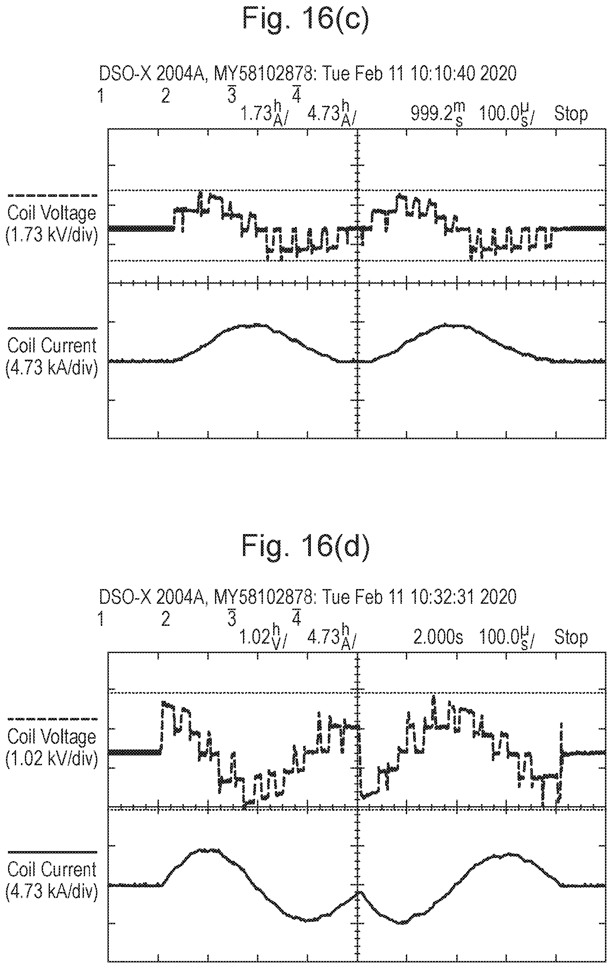

Figs. 16(a)-(d) show the measured stimulus voltage (stimulation signal) and coil current values of the stimulation circuit 2 in Fig. 14, which has a cascade of two bridge inverter stages 22. The measurements are for: (a) 2.5 kHz cosine stimulus (biphasic current); (b) 2.5 kHz sinusoidal stimulus (monophasic current); (c) two 2.5 kHz continuous sinusoidal pulses with no interval between them; and (d) two 2.5 kHz continuous cosine pulses with no interval between them with the second pulse having 180 degrees phase shift. These measurement results and comparison with the measurements in Fig. 12 prove that the cascade of two bridge inverter stages, providing 5-level PWM, produce a smoother output waveform of the magnetic field from the coil arrangement 4 than 3-levelPWM. As can be seen, the cascade embodiment produces a stimulation signal with smaller voltage steps, reducing the stress on the switches 25 and reducing high frequency harmonics in the current shape. The experimental results confirmed that the maximum delivered energy of the embodiment in Fig. 14 using a cascade of two bridge inverter stages 22 is 88% of the maximum energy of the Magstim rapid 2 option 2.

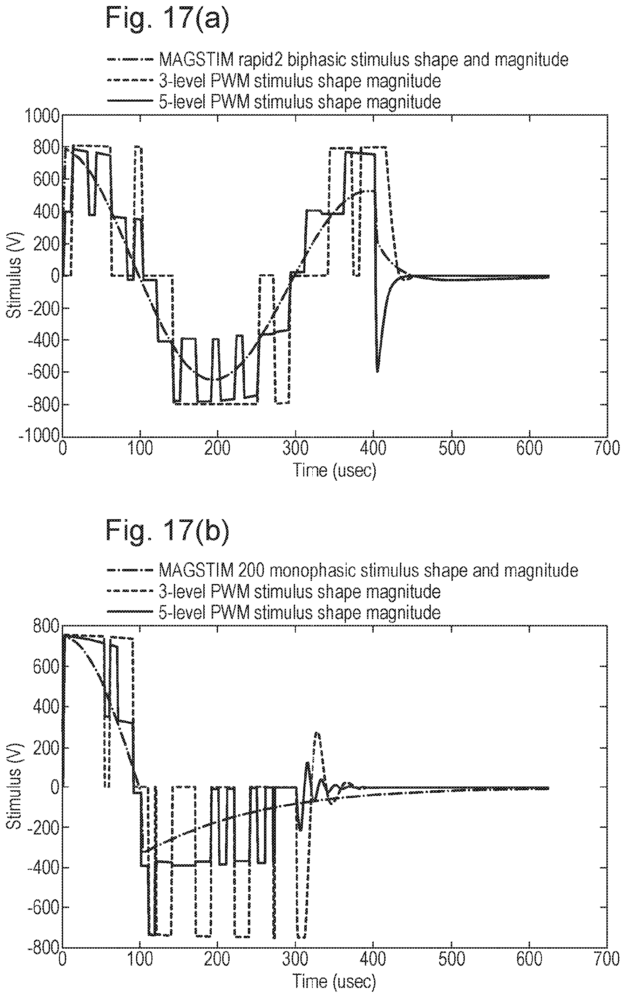

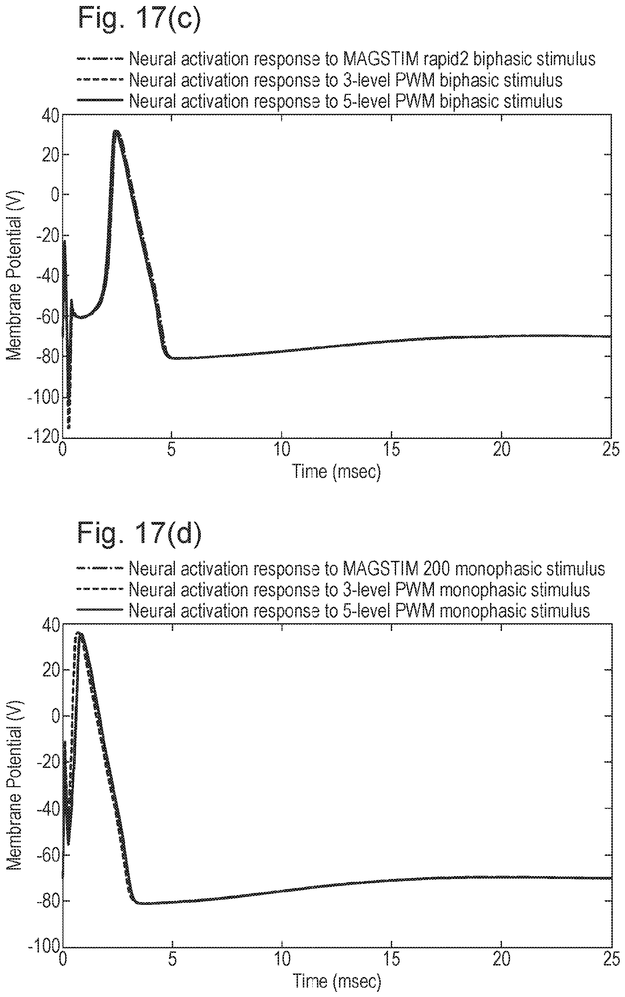

Figs. 17(a) to 17(d) compare the stimulation signals, and the neural behaviour in response to the stimulation signal, produced by existing TMS systems, MAGSTIM rapid2 (for the biphasic stimulation signal) and MAGSTIM 200 (for the monophasic stimulation signal), with the stimulation signals produced by 3-level PWM and 5-level PWM embodiments of the present stimulation circuit 2. Fig. 17 (a)-(b) show a comparison of the stimulation signal, and Fig. 17(c)-(d) compare the neural behaviour in response to the stimulation signal. The 3-level PWM embodiment is one such as shown in Fig. 7, that comprises only a single bridge inverter stage. The 5-level PWM embodiment is that of Fig. 14, with a cascade of two bridge inverter stages 22. To find neural behaviour, the Hodgkin-Huxley model is utilized. This model can describe, how action potentials in neurons are started and propagated, according to the properties of stimulus [44].

According to Fig 17 (c)- (d) all embodiments of the stimulation circuit 2 produce nearly the same neural response as the MAGSTIM rapid2 and 200, due to the filtering effects of the coil arrangement 4 and the low-pass properties of the neural tissue described above. However, the present stimulation circuit 2 has greater flexibility in the types of waveform that can be generated, and other advantages such as uniformity of pulse voltages as described above.

The ability of TMS to provide non-invasive body organ stimulation, coupled with the unmet need for better treatments of neurological disorders, motivates the development of improved capability of TMS systems to deliver more flexible patterns of stimulation. Most current research in this field focuses on the number of pulses per second, the time interval between pulses and sessions [45]. However, new devices that can produce stimuli in a wide range of flexible patterns could open new pathways for clinical neuroscience.

The programmable TMS system disclosed herein is capable of generating highly flexible stimulus waveforms in terms of both pulse shapes as well as patterns. The system uses pulse width modulation to generate flexible and stable magnetic stimuli to actuate the nervous system. The circuit working principles, designs, implementation, and experimental measurements disclosed herein demonstrate this flexibility. In particular, the specific implementations described herein achieve this performance using unipolar PWM and the 3-level or 5- level or 7- level H-bridge inverters in the TMS stimulation circuit 2. Consistent with the PWM principle, the generation of pulse trains of different widths and polarities allows the imitation of a wide range of pulses (although with the trade-off of increased harmonic distortion in some cases). The inductive property of the stimulation coil and the inherent capacitive-resistive properties of the neuron membrane help the system to filter out the high-frequency harmonics of the stimulus. The system has two key advantages. First, the production of consecutive rectangular pulses with a predetermined time interval, widths and polarities, enables the synthesis of a wide range of TMS waveforms. Consequently, the limitations of previous devices in the production of near-rectangular pulses or harmonic cosines are largely addressed. Second, by using the UPWM technique it is possible to generate different levels of stimulus voltage from the DC supply voltage. Therefore, an efficient solution to the complexity of producing different levels of stimulus current is achievable. The stimulus polarity can be adjusted in either orientation without requiring manual mechanical coil intervention.

Other advantages can be provided by specific circuit-level features of the specific implementations disclosed herein. The use of large DC capacitors allows for the generation of constant stimulus pulses and minimized stimulus voltage drop. The stabilized voltage makes it possible to generate consistent monophasic and biphasic TBS and QPS stimulation modalities. The use of parallel switches (specifically IGBTs) reduces current stress and helps to maintain all components within their SOA. Therefore, the system is arguably safer than currently available research-grade TMS systems, whilst also being more flexible. These aggregate benefits should enable new approaches to minimally invasive body organ stimulation research and therapies.

The experimental measurements demonstrate that the system is capable of generating stimuli up to 4 kHz. For a 3-level device (such as in Fig. 1), voltage and peak current values are $\pm 1000$ V and $\pm 3600$ A, respectively. For a device with two-cascaded cells (5-level PWM in Fig. 14), a voltage of $\pm 1600$ V and peak current of $\pm 6$ kA were measured. The maximum transferred energy measured in the experimental validation for 3-level and 5- level stimuli were 100.4 Joules and 250 Joules, respectively. To characterize repetitive TMS modalities, the efficiency of generating consecutive triplets and quadruplets pulses with interstimulus intervals of 1 ms was tested and verified. The proposed design enables the production of highly configurable and stable stimuli at high repetition rates.

Corresponding to the stimulation circuit described above, there is also provided a method of generating magnetic stimulation, the method comprising providing a DC supply, controlling a DC/AC inverter 20, which comprises a bridge inverter stage 22 comprising plural switch modules 24 connected in a bridge arrangement between the DC supply circuit 10 and output terminals 32, to generate a stimulation signal; and supplying the stimulation signal to a coil arrangement 4 configured to apply magnetic stimulation to a body organ 1. The method of generating magnetic stimulation may be used in a method in which the generated magnetic stimulation is applied to a body organ for diagnosis or treatment of the body organ.

**References**

[1] M. Chen, C. C. Cline and K. L. Frost, "Chapter 11 - Advances and Challenges in Transcranial Magnetic Stimulation (TMS) Research on Motor Systems," in Engineering in Medicine, Advances and Challenges, Academic Press, 2019, pp. 283-318.

[2] D. Blumberger, F. Vila-Rodriguez, K. Thorpe and K. Feffer, "Effectiveness of theta burst versus high-frequency repetitive transcranial magnetic stimulation in patients with depression (THREE-D): a randomised non-inferiority trial," The Lancet, vol. 391, no. 10131, pp. 1683-1692, APRIL 2018.

[3] L. Jean-Pascal, "Chapter 37 - Transcranial magnetic stimulation," in Handbook of Clinical Neurology, Elsevier B.V., 2019,, pp. 559-580.

[4] A. V. Peterchev, R. Jalinous and S. H. Lisanby, "A transcranial magnetic stimulator inducing near-rectangular pulses with controllable pulse width (cTMS).," IEEE TRANSACTIONS ON BIOMEDICAL ENGINEERING, vol. 55, no. 1, pp. 257-266, JANUARY 2008.

[5] A. V. Peterchev, D. L Murphy and S. H Lisanby, "Repetitive Transcranial Magnetic Stimulator with Controllable Pulse Parameters," J Neural Eng., vol. 8, no. 3, p. 036016, 2011.

[6] A. V. Peterchev, K. D'Ostilio, J. C. Rothwell and D. L. Murphy, "Controllable pulse parameter transcranial magnetic stimulator with enhanced circuit topology and pulse shaping.," Journal of neural engineering, vol. 22, no. 5, 2014.

[7] N. Gattinger, G. Moßnang and B. Gleich, "flexTMS--a novel repetitive transcranial magnetic stimulation device with freely programmable stimulus currents.," IEEE Trans Biomed Eng, vol. 59, no. 7, pp. 1962-70, Jul 2012.

[8] S. M. Goetz, M. Pfaeffl, J. Huber, M. Singer, R. Marquardt and T. Weyh, "Circuit topology and control principle for a first magnetic stimulator with fully controllable waveform," in Annual International Conference of the IEEE Engineering in Medicine and Biology Society, 2012.

[9] M. Memarian Sorkhabi, J. Frounchi, P. Shahabi and H. Veladi, "Deep-Brain Transcranial Stimulation: A Novel Approach for High 3-D Resolution," IEEE Access, vol. 5, pp. 3157-3171, 2017.

[10] K. Sang Hoon, "Chapter 7 - Pulse width modulation inverters," in Electric Motor Control, Elsevier, 2017, pp. 265-340.

[11] P. MARIAN, "CHAPTER 5 - Control of PWM Inverter-Fed Induction Motors," in Control in Power Electronics, Selected Problems, Academic Press, 2002, pp. 161-207.

[12] M. Rosa-Clot and G. M. Tina, "Chapter 3 - Introduction to PV Plants," in Submerged and Floating Photovoltaic Systems, Modelling, Design and Case Studies, academic press, 2018, pp. 33-64.

[13] N. Vázquez and J. V. López, "Inverters," in Power Electronics Handbook (Fourth Edition), Butterworth-Heinemann, 2018, pp. 289-338.

[14] B. Wu and M. Narimani, "Other Multilevel Voltage Source Inverters," in High-Power Converters and AC Drives, Wiley-IEEE Press, 2017, pp. 185-223.

[15] D. Z. Gao and K. Sun, "DC-AC inverters," in Electric Renewable Energy Systems, Academic press, 2016,, pp. 354-381.

[16] H. Niklas, "IGBT Modules in Parallel Operation with Central and Individual Driver Board," Semikron, 2017.

[17] B. B. Jayant, "Chapter 7 - Gate Drive Circuit Design," in The IGBT Device, William Andrew, 2015, pp. 193-203.

[18] F. Costa and D. Magnon, "Graphical analysis of the spectra of EMI sources in power electronics," IEEE Transactions on Power Electronics, vol. 20, no. 6, pp. 1491 - 1498, 2005.

[19] W. Gerstner, W. M. Kistler, R. Naud and L. Paninski, Neuronal Dynamics: From single neurons to networks and models of cognition, Cambridge University Press, 2014.

[20] Z.-D. Deng, S. H. Lisanby and A. V. Peterchev, "Electric field strength and focality in electroconvulsive therapy and magnetic seizure therapy: a finite element simulation study," J Neural Eng., vol. 8, no. 1, p. 016007, 2011.

[21] S. Jezernik, T. Sinkjaer and M. Morari, "Charge and energy minimization in electrical/magnetic stimulation of nervous tissue," Journal of Neural Engineering,, vol. 7, no. 4, pp. 1-15, 16 June 2010 .

[22] A. Barker, C. Garnham and I. Freeston, "Magnetic nerve stimulation: the effect of waveform on efficiency, determination of neural membrane time constants and the measurement of stimulator output.," Electroencephalogr Clin Neurophysiol Suppl, vol. 43, pp. 227-37, 1991.

[23] C. E. Corthout, A. Barker and A. Cowey, "Transcranial magnetic stimulation, Which part of the current waveform causes the stimulation?," Exp Brain Res, vol. 141, p. 128-132, September 2001.

[24] B. Wu and M. Narimani, "Two-Level Voltage Source Inverter," in High-Power Converters and AC Drives, Wiley-IEEE Press, 2017, pp. 93-117.

[25] Y. Tang and H. Ma, "Dynamic Electrothermal Model of Paralleled IGBT Modules With Unbalanced Stray Parameters," IEEE TRANSACTIONS ON POWER ELECTRONICS,, vol. VOL. 32, no. 2, pp. 1385 - 1399, 2017.

[26] H. Li, R. Yao, W. Lai, H. Ren and J. Li, "Modeling and Analysis on Overall Fatigue," IEEE TRANSACTIONS ON ELECTRON DEVICES, vol. 66, no. 3, pp. 1435-1443, 2019.

[27] M. Trivedi and K. Shenai, "Failure Mechanisms of IGBT's Under Short-Circuit," IEEE TRANSACTIONS ON POWER ELECTRONICS, vol. 14, no. 1, pp. 108-116, 1999.

[28] S. Yang, A. Bryant, P. Mawby, D. Xiang, L. Ran and P. Tavner, "An industry-based survey of reliability in power electronic converters," IEEE Transactions on Industry Applications, vol. 47, no. 3, pp. 1441-1451,, Mar. 2011.

[29] X. Perpiñà, I. Cortés, J. Urresti-Ibañez, X. Jordà and J. Rebollo, "Layout Role in Failure Physics of IGBTs Under Overloading Clamped Inductive Turnoff," IEEE TRANSACTIONS ON ELECTRON DEVICES,, vol. 60, no. 2, pp. 598-605, FEBRUARY 2013.

[30] S. Lefebvre and F. Miserey, "Analysis of CIC NPT IGBTs turn-off operations for high switching current level," IEEE Transactions on Electron Devices, vol. 46, no. 5, p. 1042-1049, May 1999.

[31] X. Perpina, J.-F. Serviere, J. Urresti-Ibanez, I. Cortes and X. Jorda, "Analysis of Clamped Inductive Turnoff Failure in Railway Traction IGBT Power Modules Under Overload Conditions," IEEE TRANSACTIONS ON INDUSTRIAL ELECTRONICS, vol. 58, no. 7, pp. 2706 - 2714, JULY 2011.

[32] C. Mauro, "Selected failure mechanisms of modern power modules," Microelectronics Reliability, vol. 42, no. 4-5, pp. 653-667, May 2002.

[33] J. Lei, D. Mingxing, W. Kexin and H. W. Gerard, "A Health Monitoring Method for Bond Wires in IGBT Modules Based on Voltage Ringing Characteristics," IEEE Transactions on Electron Devices, vol. 66, no. 9, pp. 3953 - 3960, August 2019.

[34] B. B. Jayant, "Safe Operating Area Design," in The IGBT Device, William Andrew, 2015, pp. 117-145.

[35] J. O. Nieminen, L. M. Koponen and R. J. Ilmoniemi, "Experimental Characterization of the Electric Field DistributionInduced by TMS Devices," Brain Stimulation, vol. 8, no. 3, pp. 582-589, May-June 2015.

[36] M. Memarian Sorkhabi, J. Frounchi, P. Shahabi and H. Veladi, "Measurement of transcranial magnetic stimulation resolution in 3-D spaces," Measurement, vol. 116, pp. 326-340, 2018.

[37] A. Peterchev, S. Goetz, G. Westin, B. Luber and S. Lisanby, "Pulse width dependence of motor threshold and input-output curvecharacterized with controllable pulse parameter transcranialmagnetic stimulation," Clin Neurophysiol, vol. 124, no. 7, pp. 1364-72, 2014.

[38] Q. Feng, A. D. Wu and N. Schweighofer, "Fast estimation of transcranial magnetic stimulation motor threshold," Brain stimulation, vol. 4, no. 1, pp. 50-57, 2011.

[39] Z. D. Jonker, R. v. d. Vliet, C. M. Hauwert, C. Gaiser and J. H. Tulen, "TMS motor mapping: Comparing the absolute reliability of digital reconstruction methods to the golden standard," Brain stimulation, vol. 12, no. 2, pp. 309-313, 2019.

[40] J. B. Pitcher, K. M. Ogston and T. S. Miles, "Age and sex differences in human motor cortex input-output characteristics.," The Journal of physiology, vol. 546, no. 2, pp. 605-613, 2003.

[41] C. A. Hanlon, L. T. Dowdle, D. H. Lench and T. K. Ramos, "Chapter 22 - Brain stimulation as an emerging treatment for addiction," in Cognition and Addiction, Academic press, 2020, pp. 295-302.

[42] D. Gilbert, "Chapter 4 - Therapeutic rTMS in Children," in Pediatric Brain Stimulation, Academic press, 2016, pp. 71-83.

[43] M. Hamada, Y. Terao, R. Hanajima, Y. Shirota, S. Nakatani-Enomoto, T. Furubayashi, H. Matsumoto and Y. Ugawa, "Bidirectional long-term motor cortical plasticity and metaplasticity induced by quadripulse transcranial magnetic stimulation.," J Physiol., vol. 586, no. 16, pp. 3927-47, Aug 2008.

[44] W. Drongelen, "Chapter 29 - Modeling Neural Systems: Cellular Models," in Signal Processing for Neuroscientists (Second Edition), Academic press, 2018, pp. 619-646.

[45] J. Voigt, L. Carpenter and A. Leuchter, "A systematic literature review of the clinical efficacy of repetitive transcranial magnetic stimulation (rTMS) in non-treatment resistant patients with major depressive disorder," BMC Psychiatry, vol. 19, no. 13, 2019.

**Claims**

1. A stimulation circuit (2) for generating magnetic stimulation, the stimulation circuit (2) comprising:

   a coil arrangement (4) configured to apply magnetic stimulation to a body organ;
   a DC supply circuit (10) arranged to provide a DC supply;
   a DC/AC inverter (20) arranged to receive the DC supply at input terminals (30) and generate, at output terminals (32), a stimulation signal which is supplied to the coil arrangement (4), and
   a driver circuit (41) arranged to supply pulse width modulation control signals to the switch modules (24) that are selected to control the DC/ AC inverter (20) to generate the stimulation signal with pulse width modulation of voltage, wherein:

      the DC/AC inverter (20) comprises a cascade of bridge inverter stages (22), input terminals (30) of each bridge inverter stage (22) being connected to the DC supply to receive the DC supply, output terminals (32) of the bridge inverter stages (22) being connected in series for supplying the stimulation signal with multiple voltage levels; and.
      the DC supply circuit (10) includes a capacitive energy stage (16) comprising plural isolated capacitive energy storage modules (18) each arranged to supply the DC supply, each bridge inverter stage (22) being connected to a respective isolated capacitive energy storage module (18),

   wherein the cascade of bridge inverter stages comprises a bridge inverter stage (22) comprising plural switch modules (24) connected in an H-bridge arrangement between the input terminals (30) and the output terminals (32) of the bridge inverter stage.

2. A stimulation circuit according to claim 1, wherein the driver circuit (41) is arranged to supply pulse width modulation control signals to the switch modules (24) that are selected to control the DC/AC inverter (20) to generate the stimulation signal having a controllable desired waveform, frequency and amplitude by pulse width modulation of voltage.

3. A stimulation circuit according to claim 2, wherein the driver circuit (41) is arranged to generate the pulse width modulation control signals based on a reference signal representing the desired waveform, frequency and amplitude.

4. A stimulation circuit according to claim 3, wherein the driver circuit (41) is arranged to generate the pulse width modulation control signals based on comparison of the waveforms of the reference signal and at least one carrier signal; and/or
   wherein the driver circuit (41) is arranged to accept user input specifying the desired waveform, frequency and amplitude.

5. A stimulation circuit according to claim 1, wherein each switch module (24) comprises at least two switches (25) connected in parallel.

6. A stimulation circuit according to claim 5, wherein the switch modules (24) comprise diodes (29) connected in anti-parallel across each switch (25).

7. A stimulation circuit according to claim 5 or claim 6, wherein the driver circuit (41) comprises:

   current-balancing resistors (26) connecting each switch (25) to the driver circuit (41); and/or
   transient voltage suppression diodes (43) connected across terminals of each switch (25).

8. A stimulation circuit according to any one of claims 5 to 7, wherein each switch (25) is a semiconductor switch, and/or wherein the switch modules (24) comprise snubbing circuits (27) connected in parallel with each switch (25).

9. A stimulation circuit according to any one of the preceding claims, wherein the control signals are selected to control the DC/AC inverter (20) to supply a stimulation signal with unipolar pulse width modulation of voltage.

10. A stimulation circuit according to any one of the preceding claims, wherein the control signals are selected to control the DC/ AC inverter (20) to supply a stimulation signal with multi-level pulse width modulation of voltage.

11. A stimulation circuit according to any one of the preceding claims, further comprising an output filter (46) arranged between the DC/ AC inverter (20) and the coil arrangement (4), the output filter (46) being a low-pass filter.

12. A stimulation circuit according to any one of the preceding claims, wherein the driver circuit (41) is configured to apply pre-distortion to the pulse width modulation control signals, the pre-distortion chosen to correct for distortion to the stimulation signal caused by the DC/ AC inverter (20) and/or the output filter (46), if present.

13. A stimulation circuit according to any one of the preceding claims, wherein the pulse width modulation has an average switching frequency of at least 1 kHz, preferably at least 10 kHz.

14. A stimulation circuit according to any one of the preceding claims, wherein the coil arrangement (4) has an inductance of at most 32 pH.

15. A stimulation circuit according to any one of the preceding claims, wherein the stimulation signal which has a peak current of at least 500 A and/or has a peak voltage of at least 200 V.

**Patentansprüche**

1. Eine Stimulationsschaltung (2) zum Erzeugen magnetischer Stimulation, wobei die Stimulationsschaltung (2) Folgendes beinhaltet:

   eine Spulenanordnung (4), die dazu konfiguriert ist, magnetische Stimulation auf ein Körperorgan anzuwenden;
   eine Gleichstrom-Versorgungsschaltung (10), die dazu angeordnet ist, eine Gleichstromversorgung bereitzustellen;
   einen Gleichstrom-Wechselstrom-Umrichter (20), der dazu angeordnet ist, die Gleichstromversorgung an Eingangsanschlüssen (30) aufzunehmen und an Ausgangsanschlüssen (32) ein Stimulationssignal zu erzeugen, das der Spulenanordnung (4) zugeführt wird,
   und
   eine Treiberschaltung (41), die dazu angeordnet ist, den Schaltermodulen (24), die ausgewählt sind, um den Gleichstrom-Wechselstrom-Umrichter (20) zu steuern, Pulsweitenmodulations-Steuersignale zuzuführen, um das Stimulationssignal mit Pulsweitenmodulation der Spannung zu erzeugen, wobei:

      der Gleichstrom-Wechselstrom-Umrichter (20) eine Kaskade von Brückenumrichterstufen (22) beinhaltet, Eingangsanschlüsse (30) jeder Brückenumrichterstufe (22) mit der Gleichstromversorgung verbunden sind, um die Gleichstromversorgung aufzunehmen, Ausgangsanschlüsse (32) der Brückenumrichterstufen (22) in Reihe geschaltet sind, um das Stimulationssignal mit mehreren Spannungspegeln zuzuführen; und die Gleichstromversorgungsschaltung (10) eine kapazitive Energiestufe (16) umfasst, die mehrere isolierte kapazitive Energiespeichermodule (18) beinhaltet, die jeweils dazu angeordnet sind, die Gleichstromversorgung zuzuführen, wobei jede Brückenumrichterstufe (22) mit einem jeweiligen isolierten kapazitiven Energiespeichermodul (18) verbunden ist, wobei die Kaskade von Brückenumrichterstufen eine Brückenumrichterstufe (22) beinhaltet, die mehrere Schaltermodule (24) beinhaltet, die in einer H-Brückenanordnung zwischen den Eingangsanschlüssen (30) und den Ausgangsanschlüssen (32) der Brückenumrichterstufe angeordnet und damit verbunden sind.

2. Stimulationsschaltung gemäß Anspruch 1, wobei die Treiberschaltung (41) dazu angeordnet ist, den Schaltermodulen (24), die ausgewählt sind, um den Gleichstrom-Wechselstrom-Umrichter (20) zu steuern, Pulsweitenmodulations-Steuersignale zuzuführen, um das Stimulationssignal durch Pulsweitenmodulation der Spannung mit einer steuerbaren gewünschten Wellenform, Frequenz und Amplitude zu erzeugen.

3. Stimulationsschaltung gemäß Anspruch 2, wobei die Treiberschaltung (41) dazu angeordnet ist, die Pulsweitenmodulations-Steuersignale basierend auf einem die gewünschte Wellenform, Frequenz und Amplitude repräsentierenden Referenzsignal zu erzeugen.

4. Stimulationsschaltung gemäß Anspruch 3, wobei die Treiberschaltung (41) dazu angeordnet ist, die Pulsweitenmodulations-Steuersignale basierend auf einem Vergleich der Wellenformen des Referenzsignals und mindestens eines Trägersignals zu erzeugen; und/oder
wobei die Treiberschaltung (41) dazu angeordnet ist, Benutzereingaben entgegenzunehmen, die die gewünschte Wellenform, Frequenz und Amplitude angeben.

5. Stimulationsschaltung gemäß Anspruch 1, wobei jedes Schaltermodul (24) mindestens zwei parallel geschaltete Schalter (25) beinhaltet.

6. Stimulationsschaltung gemäß Anspruch 5, wobei die Schaltermodule (24) Dioden (29) beinhalten, die antiparallel über jeden Schalter (25) hinweg angeschlossen sind.

7. Stimulationsschaltung gemäß Anspruch 5 oder Anspruch 6, wobei die Treiberschaltung (41) Folgendes beinhaltet:

   Stromausgleichswiderstände (26), die jeden Schalter (25) mit der Treiberschaltung (41) verbinden; und/oder
   Überspannungsschutzdioden (43), die über Anschlüsse jedes Schalters (25) hinweg angeschlossen sind.

8. Stimulationsschaltung gemäß einem der Ansprüche 5 bis 7, wobei jeder Schalter (25) ein Halbleiterschalter ist und/oder wobei die Schaltermodule (24) Entstörschaltungen (27) beinhalten, die mit jedem Schalter (25) parallel geschaltet sind.

9. Stimulationsschaltung gemäß einem der vorhergehenden Ansprüche, wobei die Steuersignale ausgewählt sind, um den Gleichstrom-Wechselstrom-Umrichter (20) zu steuern, um ein Stimulationssignal mit unipolarer Pulsweitenmodulation der Spannung zuzuführen.

10. Stimulationsschaltung gemäß einem der vorhergehenden Ansprüche, wobei die Steuersignale ausgewählt sind, um den Gleichstrom-Wechselstrom-Umrichter (20) zu steuern, um ein Stimulationssignal mit mehrstufiger Pulsweitenmodulation der Spannung zuzuführen.

11. Stimulationsschaltung gemäß einem der vorhergehenden Ansprüche, ferner beinhaltend einen Ausgangsfilter (46), der zwischen dem Gleichstrom-Wechselstrom-Umrichter (20) und der Spulenanordnung (4) angeordnet ist, wobei der Ausgangsfilter (46) ein Tiefpassfilter ist.

12. Stimulationsschaltung gemäß einem der vorhergehenden Ansprüche, wobei die Treiberschaltung (41) dazu konfiguriert ist, Vorverzerrung auf die Pulsweitenmodulations-Steuersignale anzuwenden, wobei die Vorverzerrung gewählt wird, um durch den Gleichstrom-Wechselstrom-Umrichter (20) und/oder den Ausgangsfilter (46), sofern vorhanden, verursachte Verzerrung des Stimulationssignals auszugleichen.

13. Stimulationsschaltung gemäß einem der vorhergehenden Ansprüche, wobei die Pulsweitenmodulation eine durchschnittliche Schaltfrequenz von mindestens 1 kHz, vorzugsweise mindestens 10 kHz aufweist.

14. Stimulationsschaltung gemäß einem der vorhergehenden Ansprüche, wobei die Spulenanordnung (4) eine Induktivität von höchstens 32 pH aufweist.

15. Stimulationsschaltung gemäß einem der vorhergehenden Ansprüche, wobei das Stimulationssignal, das einen Spitzenstrom von mindestens 500 A aufweist und/oder eine Spitzenspannung von mindestens 200 V aufweist.

**Revendications**

1. Un circuit de stimulation (2) pour générer une stimulation magnétique, le circuit de stimulation (2) comprenant :

   un agencement de bobine (4) configuré pour appliquer une stimulation magnétique à un organe corporel ;
   un circuit d'alimentation CC (10) agencé pour fournir une alimentation CC ;
   un onduleur CC/CA (20) agencé pour recevoir l'alimentation CC au niveau de bornes d'entrée (30) et générer, au niveau de bornes de sortie (32), un signal de stimulation qui est fourni à l'agencement de bobine (4), et
   un circuit d'attaque (41) agencé pour fournir des signaux de commande à modulation de largeur d'impulsions aux modules de commutateur (24) qui sont sélectionnés pour commander l'onduleur CC/CA (20) afin de générer le signal de stimulation avec une modulation de largeur d'impulsions de tension, dans lequel :

   l'onduleur CC/CA (20) comprend une cascade d'étages onduleurs en pont (22), des bornes d'entrée (30) de chaque étage onduleur en pont (22) étant connectées à l'alimentation CC afin de recevoir l'alimentation CC, des bornes de sortie (32) des étages onduleurs en pont (22) étant connectées en série pour fournir le signal de stimulation avec de multiples niveaux de tension ; et
   le circuit d'alimentation CC (10) inclut un étage d'énergie capacitif (16) comprenant plusieurs modules de stockage d'énergie capacitif isolés (18) chacun agencé pour fournir l'alimentation CC, chaque étage onduleur en pont (22) étant connecté à un module de stockage d'énergie capacitif isolé (18) respectif, dans lequel la cascade d'étages onduleurs en pont comprend un étage onduleur en pont (22) comprenant plusieurs modules de commutateur (24) connectés en un agencement en pont en H entre les bornes d'entrée (30) et les bornes de sortie (32) de l'étage onduleur en pont.

2. Un circuit de stimulation selon la revendication 1, dans lequel le circuit d'attaque (41) est agencé pour fournir des signaux de commande à modulation de largeur d'impulsions aux modules de commutateur (24) qui sont sélectionnés pour commander l'onduleur CC/CA (20) afin de générer le signal de stimulation ayant une forme d'onde, une fréquence et une amplitude souhaitées pouvant être commandées par modulation de largeur d'impulsions de tension.

3. Un circuit de stimulation selon la revendication 2, dans lequel le circuit d'attaque (41) est agencé pour générer les signaux de commande à modulation de largeur d'impulsions sur la base d'un signal de référence représentant la forme d'onde, la fréquence et l'amplitude souhaitées.

4. Un circuit de stimulation selon la revendication 3, dans lequel le circuit d'attaque (41) est agencé pour générer les signaux de commande à modulation de largeur d'impulsions sur la base d'une comparaison des formes d'onde du signal de référence et d'au moins un signal porteur ; et/ou
   dans lequel le circuit d'attaque (41) est agencé pour accepter une entrée utilisateur spécifiant la forme d'onde, la fréquence et l'amplitude souhaitées.

5. Un circuit de stimulation selon la revendication 1, dans lequel chaque module de commutateur (24) comprend au moins deux commutateurs (25) connectés en parallèle.

6. Un circuit de stimulation selon la revendication 5, dans lequel les modules de commutateur (24) comprennent des diodes (29) connectées en antiparallèle de part en part de chaque commutateur (25).

7. Un circuit de stimulation selon la revendication 5 ou la revendication 6, dans lequel le circuit d'attaque (41) comprend :

   des résistances d'équilibrage de courant (26) connectant chaque commutateur (25) au circuit d'attaque (41) ; et/ou
   des diodes de suppression des tensions transitoires (43) connectées aux bornes de chaque commutateur (25).

8. Un circuit de stimulation selon l'une quelconque des revendications 5 à 7, dans lequel chaque commutateur (25) est un commutateur à semi-conducteur, et/ou dans lequel les modules de commutateur (24) comprennent des circuits d'amortissement (27) connectés en parallèle avec chaque commutateur (25).

9. Un circuit de stimulation selon l'une quelconque des revendications précédentes, dans lequel les signaux de commande sont sélectionnés pour commander l'onduleur CC/CA (20) afin de fournir un signal de stimulation avec une modulation de largeur d'impulsions unipolaire de tension.

**10.** Un circuit de stimulation selon l'une quelconque des revendications précédentes, dans lequel les signaux de commande sont sélectionnés pour commander l'onduleur CC/CA (20) afin de fournir un signal de stimulation avec une modulation de largeur d'impulsions multi-niveaux de tension.

**11.** Un circuit de stimulation selon l'une quelconque des revendications précédentes, comprenant en outre un filtre de sortie (46) agencé entre l'onduleur CC/CA (20) et l'agencement de bobine (4), le filtre de sortie (46) étant un filtre passe-bas.

**12.** Un circuit de stimulation selon l'une quelconque des revendications précédentes, dans lequel le circuit d'attaque (41) est configuré pour appliquer une pré-distorsion aux signaux de commande à modulation de largeur d'impulsions, la pré-distorsion étant choisie pour corriger une distorsion du signal de stimulation causée par l'onduleur CC/CA (20) et/ou le filtre de sortie (46), s'il est présent.

**13.** Un circuit de stimulation selon l'une quelconque des revendications précédentes, dans lequel la modulation de largeur d'impulsions a une fréquence de commutation moyenne d'au moins 1 kHz, de préférence d'au moins 10 kHz.

**14.** Un circuit de stimulation selon l'une quelconque des revendications précédentes, dans lequel l'agencement de bobine (4) a une inductance de 32 pH au plus.

**15.** Un circuit de stimulation selon l'une quelconque des revendications précédentes, dans lequel le signal de stimulation qui a un courant de crête d'au moins 500 A et/ou a une tension de crête d'au moins 200 V.

Fig. 1

## Fig. 2(a)

## Fig. 2(a)

# Fig. 3

# Fig. 4

Fig. 5(a)

Fig. 5(b)

Fig. 5(c)

Fig. 5(d)

# Fig. 5(e)

# Fig. 5(f)

# Fig. 5(g)

# Fig. 5(h)

# Fig. 6

Fig. 7

## Fig. 8

## Fig. 9

Fig. 10(a)

Fig. 10(b)

Fig. 11

# Fig. 12(a)

------- Coil Voltage (500 V / div
——— Coil Current (1 kA / div)

# Fig. 12(b)

------- Coil Voltage (500 V / div
——— Coil Current (1 kA / div)

# Fig. 12(c)

------- Coil Voltage (500 V / div
——— Coil Current (1 kA / div)

# Fig. 12(d)

------- Coil Voltage (500 V / div
——— Coil Current (1 kA / div)

## Fig. 12(e)

Time

------- Electric Field (1 (V/cm)/div)
——— Membrane Potential
      Change (ΔV) (200 mV / div)

## Fig. 12(f)

Time

------- Electric Field (1 (V/cm)/div)
——— Membrane Potential
      Change (ΔV) (200 mV / div)

## Fig. 12(g)

Time

------- Electric Field (1 (V/cm)/div)
——— Membrane Potential
      Change (ΔV) (200 mV / div)

## Fig. 12(h)

Time

------- Electric Field (1 (V/cm)/div)
——— Membrane Potential
      Change (ΔV) (200 mV / div)

## Fig. 13(a)

DSO-X 2004A, MY58102878:

------- Coil voltage    ----- Coil Current    ——— Membrane potential change

## Fig. 13(b)

DSO-X 2004A, MY58102878:

------- Coil voltage    ----- Coil Current    ——— Membrane potential change

Fig. 14

Fig. 15(a)

Fig. 15(b)

# Fig. 16(a)

DSO-X 2004A, MY58102878: Thu Feb 06 11:02:09 2020

1  2  $\overline{3}$  $\overline{4}$

604V/  1.85$\frac{h}{A}$/  1.000s  50.00$\frac{\mu}{s}$/  Stop

Coil Voltage
(604 V/div)

Coil Current
(1.85 kA/div)

# Fig. 16(b)

DSO-X 2004A, MY58102878: Thu Feb 06 10:27:19 2020

1  2  $\overline{3}$  $\overline{4}$

480V/  1.84$\frac{h}{A}$/  999.9$\frac{m}{s}$  50.00$\frac{\mu}{s}$/  Stop

Coil Voltage
(480 kV/div)

Coil Current
(1.84 kA/div)

## Fig. 16(c)

DSO-X 2004A, MY58102878: Tue Feb 11 10:10:40 2020

Coil Voltage
(1.73 kV/div)

Coil Current
(4.73 kA/div)

## Fig. 16(d)

DSO-X 2004A, MY58102878: Tue Feb 11 10:32:31 2020

Coil Voltage
(1.02 kV/div)

Coil Current
(4.73 kA/div)

## Fig. 17(a)

## Fig. 17(b)

# Fig. 17(c)

Legend:
- ·—·—· Neural activation response to MAGSTIM rapid2 biphasic stimulus
- ------ Neural activation response to 3-level PWM biphasic stimulus
- ——— Neural activation response to 5-level PWM biphasic stimulus

Y-axis: Membrane Potential (V)
X-axis: Time (msec)

# Fig. 17(d)

Legend:
- ·—·—· Neural activation response to MAGSTIM 200 monophasic stimulus
- ------ Neural activation response to 3-level PWM monophasic stimulus
- ——— Neural activation response to 5-level PWM monophasic stimulus

Y-axis: Membrane Potential (V)
X-axis: Time (msec)

# Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013030239 A1

### Non-patent literature cited in the description

- Chapter 11 - Advances and Challenges in Transcranial Magnetic Stimulation (TMS) Research on Motor Systems. **M. CHEN** ; **C. C. CLINE** ; **K. L. FROST**. Engineering in Medicine, Advances and Challenges. Academic Press, 2019, 283-318
- **D. BLUMBERGER** ; **F. VILA-RODRIGUEZ** ; **K. THORPE** ; **K. FEFFER**. Effectiveness of theta burst versus high-frequency repetitive transcranial magnetic stimulation in patients with depression (THREE-D): a randomised non-inferiority trial. *The Lancet*, April 2018, vol. 391 (10131), 1683-1692
- Chapter 37 - Transcranial magnetic stimulation. **L. JEAN-PASCAL**. Handbook of Clinical Neurology. Elsevier B.V., 2019, 559-580
- **A. V. PETERCHEV** ; **R. JALINOUS** ; **S. H. LISANBY**. A transcranial magnetic stimulator inducing near-rectangular pulses with controllable pulse width (cTMS).. *IEEE TRANSACTIONS ON BIOMEDICAL ENGINEERING*, January 2008, vol. 55 (1), 257-266
- **A. V. PETERCHEV** ; **D. L MURPHY** ; **S. H LISANBY**. Repetitive Transcranial Magnetic Stimulator with Controllable Pulse Parameters. *J Neural Eng.*, 2011, vol. 8 (3), 036016
- **A. V. PETERCHEV** ; **K. D'OSTILIO** ; **J. C. ROTHWELL** ; **D. L. MURPHY**. Controllable pulse parameter transcranial magnetic stimulator with enhanced circuit topology and pulse shaping.. *Journal of neural engineering*, 2014, vol. 22 (5)
- **N. GATTINGER** ; **G. MOßNANG** ; **B. GLEICH**. flexTMS--a novel repetitive transcranial magnetic stimulation device with freely programmable stimulus currents.. *IEEE Trans Biomed Eng*, July 2012, vol. 59 (7), 1962-70
- **S. M. GOETZ** ; **M. PFAEFFL** ; **J. HUBER** ; **M. SINGER** ; **R. MARQUARDT** ; **T. WEYH**. Circuit topology and control principle for a first magnetic stimulator with fully controllable waveform. *Annual International Conference of the IEEE Engineering in Medicine and Biology Society*, 2012
- **M. MEMARIAN SORKHABI** ; **J. FROUNCHI** ; **P. SHAHABI** ; **H. VELADI**. Deep-Brain Transcranial Stimulation: A Novel Approach for High 3-D Resolution. *IEEE Access*, 2017, vol. 5, 3157-3171
- Chapter 7 - Pulse width modulation inverters. **K. SANG HOON**. Electric Motor Control. Elsevier, 2017, 265-340
- CHAPTER 5 - Control of PWM Inverter-Fed Induction Motors. **P. MARIAN**. Control in Power Electronics, Selected Problems. Academic Press, 2002, 161-207
- Chapter 3 - Introduction to PV Plants. **M. ROSA-CLOT** ; **G. M. TINA**. Submerged and Floating Photovoltaic Systems, Modelling, Design and Case Studies. academic press, 2018, 33-64
- Inverters. **N. VÁZQUEZ** ; **J. V. LÓPEZ**. Power Electronics Handbook. Butterworth-Heinemann, 2018, 289-338
- Other Multilevel Voltage Source Inverters. **B. WU** ; **M. NARIMANI**. High-Power Converters and AC Drives. Wiley-IEEE Press, 2017, 185-223
- DC-AC inverters. **D. Z. GAO** ; **K. SUN**. Electric Renewable Energy Systems. Academic press, 2016, 354-381
- **H. NIKLAS**. IGBT Modules in Parallel Operation with Central and Individual Driver Board. *Semikron*, 2017
- Chapter 7 - Gate Drive Circuit Design. **B. B. JAYANT**. The IGBT Device. William Andrew, 2015, 193-203
- **F. COSTA** ; **D. MAGNON**. Graphical analysis of the spectra of EMI sources in power electronics. *IEEE Transactions on Power Electronics*, 2005, vol. 20 (6), 1491-1498
- **W. GERSTNER** ; **W. M. KISTLER** ; **R. NAUD** ; **L. PANINSKI**. Neuronal Dynamics: From single neurons to networks and models of cognition. Cambridge University Press, 2014
- **Z.-D. DENG** ; **S. H. LISANBY** ; **A. V. PETERCHEV**. Electric field strength and focality in electroconvulsive therapy and magnetic seizure therapy: a finite element simulation study. *J Neural Eng.*, 2011, vol. 8 (1), 016007
- **S. JEZERNIK** ; **T. SINKJAER** ; **M. MORARI**. Charge and energy minimization in electrical/magnetic stimulation of nervous tissue. *Journal of Neural Engineering*, 16 June 2010, vol. 7 (4), 1-15

- **A. BARKER** ; **C. GARNHAM** ; **I. FREESTON**. Magnetic nerve stimulation: the effect of waveform on efficiency, determination of neural membrane time constants and the measurement of stimulator output.. *Electroencephalogr Clin Neurophysiol Suppl*, 1991, vol. 43, 227-37
- **C. E. CORTHOUT** ; **A. BARKER** ; **A. COWEY**. Transcranial magnetic stimulation, Which part of the current waveform causes the stimulation?. *Exp Brain Res*, September 2001, vol. 141, 128-132
- Two-Level Voltage Source Inverter. **B. WU** ; **M. NARIMANI**. High-Power Converters and AC Drives. Wiley-IEEE Press, 2017, 93-117
- **Y. TANG** ; **H. MA**. Dynamic Electrothermal Model of Paralleled IGBT Modules With Unbalanced Stray Parameters. *IEEE TRANSACTIONS ON POWER ELECTRONICS*, 2017, vol. 32 (2), 1385-1399
- **H. LI** ; **R. YAO** ; **W. LAI** ; **H. REN** ; **J. LI**. Modeling and Analysis on Overall Fatigue. *IEEE TRANSACTIONS ON ELECTRON DEVICES*, 2019, vol. 66 (3), 1435-1443
- **M. TRIVEDI** ; **K. SHENAI**. Failure Mechanisms of IGBT's Under Short-Circuit. *IEEE TRANSACTIONS ON POWER ELECTRONICS*, 1999, vol. 14 (1), 108-116
- **S. YANG** ; **A. BRYANT** ; **P. MAWBY** ; **D. XIANG** ; **L. RAN** ; **P. TAVNER**. An industry-based survey of reliability in power electronic converters. *IEEE Transactions on Industry Applications*, March 2011, vol. 47 (3), 1441-1451
- **X. PERPIÑÀ** ; **I. CORTÉS** ; **J. URRESTI-IBAÑEZ** ; **X. JORDÀ** ; **J. REBOLLO**. Layout Role in Failure Physics of IGBTs Under Overloading Clamped Inductive Turnoff. *IEEE TRANSACTIONS ON ELECTRON DEVICES*, February 2013, vol. 60 (2), 598-605
- **S. LEFEBVRE** ; **F. MISEREY**. Analysis of CIC NPT IGBTs turn-off operations for high switching current level. *IEEE Transactions on Electron Devices*, May 1999, vol. 46 (5), 1042-1049
- **X. PERPINA** ; **J.-F. SERVIERE** ; **J. URRESTI-IBANEZ** ; **I. CORTES** ; **X. JORDA**. Analysis of Clamped Inductive Turnoff Failure in Railway Traction IGBT Power Modules Under Overload Conditions. *IEEE TRANSACTIONS ON INDUSTRIAL ELECTRONICS*, July 2011, vol. 58 (7), 2706-2714
- **C. MAURO**. Selected failure mechanisms of modern power modules. *Microelectronics Reliability*, May 2002, vol. 42 (4-5), 653-667
- **J. LEI** ; **D. MINGXING** ; **W. KEXIN** ; **H. W. GERARD**. A Health Monitoring Method for Bond Wires in IGBT Modules Based on Voltage Ringing Characteristics. *IEEE Transactions on Electron Devices*, August 2019, vol. 66 (9), 3953-3960
- Safe Operating Area Design. **B. B. JAYANT**. The IGBT Device. William Andrew, 2015, 117-145
- **J. O. NIEMINEN** ; **L. M. KOPONEN** ; **R. J. ILMONIEMI**. Experimental Characterization of the Electric Field Distribution Induced by TMS Devices. *Brain Stimulation*, May 2015, vol. 8 (3), 582-589
- **M. MEMARIAN SORKHABI** ; **J. FROUNCHI** ; **P. SHAHABI** ; **H. VELADI**. Measurement of transcranial magnetic stimulation resolution in 3-D spaces. *Measurement*, 2018, vol. 116, 326-340
- **A. PETERCHEV** ; **S. GOETZ** ; **G. WESTIN** ; **B. LUBER** ; **S. LISANBY**. Pulse width dependence of motor threshold and input-output curve characterized with controllable pulse parameter transcranial magnetic stimulation. *Clin Neurophysiol*, 2014, vol. 124 (7), 1364-72
- **Q. FENG** ; **A. D. WU** ; **N. SCHWEIGHOFER**. Fast estimation of transcranial magnetic stimulation motor threshold. *Brain stimulation*, 2011, vol. 4 (1), 50-57
- **Z. D. JONKER** ; **R. V. D. VLIET** ; **C. M. HAUWERT** ; **C. GAISER** ; **J. H. TULEN**. TMS motor mapping: Comparing the absolute reliability of digital reconstruction methods to the golden standard. *Brain stimulation*, 2019, vol. 12 (2), 309-313
- **J. B. PITCHER** ; **K. M. OGSTON** ; **T. S. MILES**. Age and sex differences in human motor cortex input-output characteristics. *The Journal of physiology*, 2003, vol. 546 (2), 605-613
- Chapter 22 - Brain stimulation as an emerging treatment for addiction. **C. A. HANLON** ; **L. T. DOWDLE** ; **D. H. LENCH** ; **T. K. RAMOS**. Cognition and Addiction. Academic press, 2020, 295-302
- Chapter 4 - Therapeutic rTMS in Children. **D. GILBERT**. Pediatric Brain Stimulation. Academic press, 2016, 71-83
- **M. HAMADA** ; **Y. TERAO** ; **R. HANAJIMA** ; **Y. SHIROTA** ; **S. NAKATANI-ENOMOTO** ; **T. FURU-BAYASHI** ; **H. MATSUMOTO** ; **Y. UGAWA**. Bidirectional long-term motor cortical plasticity and metaplasticity induced by quadripulse transcranial magnetic stimulation.. *J Physiol.*, August 2008, vol. 586 (16), 3927-47
- Chapter 29 - Modeling Neural Systems: Cellular Models. **W. DRONGELEN**. Signal Processing for Neuroscientists (Second Edition). Academic press, 2018, 619-646
- **J. VOIGT** ; **L. CARPENTER** ; **A. LEUCHTER**. A systematic literature review of the clinical efficacy of repetitive transcranial magnetic stimulation (rTMS) in non-treatment resistant patients with major depressive disorder. *BMC Psychiatry*, 2019, vol. 19 (13)